(19) Europäisches Patentamt
European Patent Office
Office européen des brevets

(11) EP 4 471 790 B1

(12) EUROPEAN PATENT SPECIFICATION

(45) Date of publication and mention
of the grant of the patent:
01.04.2026 Bulletin 2026/14

(21) Application number: 24172137.2

(22) Date of filing: 24.04.2024

(51) International Patent Classification (IPC):
G16H 10/40 (2018.01)      G16H 50/20 (2018.01)

(52) Cooperative Patent Classification (CPC):
G16H 50/20; G16H 10/40; G16H 15/00

(54) **SYSTEM AND METHOD FOR DETERMINING MICROBIOME FROM HOST METABOLOME USING A MACHINE LEARNING MODEL**

SYSTEM UND VERFAHREN ZUR BESTIMMUNG DES MIKROBIOMS AUS EINEM HOST-METABOLOM UNTER VERWENDUNG EINES MASCHINENLERNMODELLS

SYSTÈME ET PROCÉDÉ DE DÉTERMINATION DE MICROBIOME À PARTIR D'UN MÉTABOLOME HÔTE À L'AIDE D'UN MODÈLE D'APPRENTISSAGE AUTOMATIQUE

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: 02.06.2023 IN 202341038067

(43) Date of publication of application:
04.12.2024 Bulletin 2024/49

(73) Proprietors:
• MicrobioTx Health Private Limited
560034 Bangalore Karnataka (IN)
• National Institute of Science Education and Research
752050 Bhubaneswar Odisha (IN)

(72) Inventor: AICH, Palok
752050 Bhubaneswar (IN)

(74) Representative: ip21 Ltd
Central Formalities Department
Suite 2
The Old Dairy
Elm Farm Business Park
Wymondham
Norwich, Norfolk NR18 0SW (GB)

(56) References cited:
US-A1- 2019 127 781

• HAN SHUO ET AL: "A metabolomics pipeline for the mechanistic interrogation of the gut microbiome", NATURE,, vol. 595, no. 7867, 14 July 2021 (2021-07-14), pages 415 - 420, XP037507988, DOI: 10.1038/S41586-021-03707-9
• NOECKER CECILIA ET AL: "High-resolution characterization of the human microbiome", TRANSLATIONAL RESEARCH, ELSEVIER, AMSTERDAM, NL, vol. 179, 25 July 2016 (2016-07-25), pages 7 - 23, XP029850261, ISSN: 1931-5244, DOI: 10.1016/J.TRSL.2016.07.012
• NGUYEN QUANG P. ET AL: "Associations between the gut microbiome and metabolome in early life", BMC MICROBIOLOGY, vol. 21, no. 1, 28 August 2021 (2021-08-28), GB, XP093207147, ISSN: 1471-2180, Retrieved from the Internet <URL:https://link.springer.com/article/10.1186/s12866-021-02282-3/fulltext.html> DOI: 10.1186/s12866-021-02282-3
• YIN XIAOCHEN ET AL: "A Comparative Evaluation of Tools to Predict Metabolite Profiles From Microbiome Sequencing Data", FRONTIERS IN MICROBIOLOGY, vol. 11, 4 December 2020 (2020-12-04), Lausanne, XP093207150, ISSN: 1664-302X, DOI: 10.3389/fmicb.2020.595910
• EFRAT MULLER, YADID M. ALGAVI , ELHANAN BORENSTEIN: "The gut microbiome-metabolome dataset collection: a curated resource for integrative meta-analysis", NPJ BIOFILMS AND MICROBIOMES, vol. 8, no. 1, 15 October 2022 (2022-10-15), XP093207152, ISSN: 2055-5008, DOI: 10.1038/s41522-022-00345-5

## Description

*Technical Field*

The embodiments herein generally relate to microbiome analysis for disease identification, more particularly, a system and method for determining microbiome from blood metabolites (metabolome) using artificial intelligence (AI) techniques and a machine learning model.

*Description of the Related Art*

Timely detection of a disease can significantly increases the chances of a successful cure or longer survival. For many chronic diseases, there are specialized detection methods available that can diagnose the disease in its asymptomatic stage, before any signs or symptoms appear. However, these existing methods may not always detect the disease early enough for an effective intervention. Additionally, most of the current detection methods are invasive and can be quite expensive.

One promising approach to developing a sensitive tool and effective means of health intervention is by utilizing an intrinsic component of the human body, such as the gut microbiota. The gut microbiota is a diverse community of microorganisms that colonize in the gastrointestinal tract, and its bacteria play a multifunctional role in the body. These functions include (a) producing essential nutrients and co-metabolizing food, (b) preventing bacterial overgrowth and infection, and (c) influencing central physiological functions such as the development of lymphatic tissue, the induction of mucosal tolerance, angiogenesis, and fat storage.

Activating the innate immune system, rather than directly attacking the microbe or other disease-causing agents, can be an effective way to treat various diseases. In this regard, probiotics represent a promising avenue for creating novel antibiotics that induce innate immune responses to combat diseases. To achieve this, it is crucial to characterize the microbiome for individuals, which include bacteria, viruses, fungi, and parasites, as it can serve as a unique fingerprint for disease detection and intervention.

The most frequently used method for analysing the gut microbiome is Next-generation sequencing, which involves taking a faecal sample and reporting on the abundances of various microbial strains found within it. However, this technique can be both expensive and time-consuming, with an average test cost ranging from $100-$200 and a waiting time of 4-8 weeks for results. Additionally, while these tests do report on microbial population characteristics, they do not capture the comprehensive metabolomic profile characteristics that are more critical determinants of human health. Moreover, collecting, transporting, and handling faecal samples is challenging, leading to multiple sample losses and operational issues globally.

Some existing approaches use machine learning models that predict gut microbiome alpha diversity from serum or blood metabolites to detect type 2 Diabetes. However, these approaches are still ineffective in terms of accurate prediction of the gut microbiome, and disease correlation. Further, these approaches are only limited to predicting diversity and only for disease area (rather for example, mental and physical wellness).

HAN SHUO ET AL, "A metabolomics pipeline for the mechanistic interrogation of the gut microbiome", NATURE, vol. 595, no. 7867, 14 July 2021, pages 415-420, disclose a microbiome-focused mass-spectrometry pipeline to accelerate identification of microbiota-dependent metabolites in diverse sample types. They report the metabolic profiles of 178 gut microorganism strains using our library of 833 metabolites. Using this metabolomics resource, they establish deviations in the relationships between phylogeny and metabolism.

Therefore, there arises a need to address the aforementioned technical drawbacks in existing methods in determining the gut microbiome effectively at low cost and less time without any sample loss to accurately detect early onset of a disease and/or develop a personalized probiotic-based solution.

## SUMMARY OF THE INVENTION

The Invention is defined by the appended independent claims. Preferred embodiments are defined in the dependent claims

## SUMMARY OF RELATED DISCLOSURE

In view of a foregoing, an embodiment herein provides a system for determining a microbiome profile from the metabolome of a host. The system includes (i) an analytical device that is configured to analyze a sample and obtain spectral data of one or more metabolites present in the sample. The sample is collected from the host on a sample collection device; (ii) and a microbiome profile determination server that is communicatively connected with the analytical device and comprises a memory; and a processor in communication with the memory. The processor is configured to: (a) receive the spectral data associated with the sample from the analytical device; (b) generate metabolome data associated with the sample by detecting peaks associated with the one or more metabolites in the spectral data, and matching values of the peaks with theoretical peak values of known metabolites in a public database. The peaks values include at least one of peak intensity, mass-to-charge (m/z), peak area, or full width at half maximum (FWHM) of peak. The metabolome data includes one or more metabolites in the sample with relative abundance; and (d) predict, using a machine learning model, the microbiome profile of the host by extracting one or more features of the metabolome data and correlating the one or more features with learned association patterns to predict the microbiome profile. The microbiome profile includes at least one of phylum, genus or species level information on microbial population associated with the host with relative abundance.

**In** some embodiments, the host includes at least one of human, animal, or microbes. **In** some embodiments, the sample includes at least one of whole blood, serum, plasma, faeces, saliva, skin tissues, microbial swabs from at least one of vaginal, oral or skin, or body fluids including tears, sweat and urine.

**In** some embodiments, the analytical device is a liquid chromatography tandem mass spectrometry (LC-MS/MS) analyzer that obtains the mass spectral data of the sample. The mass spectral data includes information about mass-to-charge (m/z) ratios of detected ions in the sample, relative abundance or intensity of the detected ions, and fragmentation pattern of ions. The mass to charge (m/z) values of the peaks are matched with theoretical m/z values of known metabolites to generate the metabolome data.

**In** some embodiments, the one or more features of the metabolome data are extracted based on the relative abundance of the plurality of metabolites in the sample, wherein the one or more features of the metabolome data comprise a presence of particular metabolite, a concentration of metabolites, ratios of certain metabolites, temporal dynamics of metabolite concentrations, and diversity indices of metabolites.

**In** some embodiments, the machine learning model is trained by (i) performing, using a regression model, a correlation analysis between metabolome data associated with historical blood samples and microbiome data in historical faecal samples to obtain association patterns relating to historical metabolites and the corresponding microbiome. The association patterns are obtained based on high accuracy and high spearman correlation coefficient value; and (ii) training the machine learning model by mapping the historical metabolites to the corresponding microbiome based on the association patterns, thereby acquiring the learned association patterns that enable the machine learning model to predict the microbiome profile.

In some embodiments, the processor is configured to retrain the machine learning model by mapping the metabolome data associated with the sample to the microbiome profile that is predicted.

In some embodiments, the processor is configured to generate a microbiome report of the host based on the microbiome profile and send the microbiome report to a user device. The microbiome report includes at least one of phylum, genus or species level distribution of microbiome, information on harmful and helpful microbiomes with the abundance, and a firmicutes to bacteriodetes (F/B) ratio.

In one aspect, a method for determining a microbiome profile from the metabolome of a host is provided. The method includes (i) collecting, using a sample collection device, a sample from the host; (ii) obtaining, using an analytical device, spectral data of one or more metabolites present in the sample by analyzing the sample that is collected using the sample collection device; (iii) receiving, by a processor of a microbiome profile determination server, the spectral data associated with the sample from the analytical device; (iv) generating, by the processor, metabolome data associated with the sample by detecting peaks associated with the one or more metabolites in the spectral data, and matching values of the peaks with theoretical peak values of known metabolites in a public database. The peaks values include at least one of peak intensity, mass-to-charge (m/z), peak area, or full width at half maximum (FWHM) of peak. The metabolome data includes one or more metabolites in the sample with relative abundance; and (v) predicting, by the processor, the microbiome profile of the host by extracting one or more features of the metabolome data

and correlating the one or more features with learned association patterns to predict the microbiome profile using a machine learning model. The microbiome profile includes at least one of phylum, genus or species level information on microbial population associated with the host with relative abundance.

In some embodiments, the one or more features of the metabolome data are extracted based on the relative abundance of the plurality of metabolites in the sample, wherein the one or more features of the metabolome data include a presence of particular metabolite, a concentration of metabolites, ratios of certain metabolites, temporal dynamics of metabolite concentrations, and diversity indices of metabolites.

In some embodiments, the method includes generating, by the processor, a microbiome report of the host based on the microbiome profile. The microbiome report includes phylum, genus or species level distribution of microbiome, information on harmful and helpful microbiomes with the abundance, and a firmicutes to bacteriodetes (F/B) ratio.

The system provides a non-invasive metabolite-based microbiome predictive tool. With the system, a blood on card based sensitive metabolome data (metabolomic signatures or metabolite markers) is developed and the microbiome profile of the host is predicted accurately from the metabolome data. Hence, the predicted microbiome profile establishes the health status; mineral and vitamin deficiencies; overall gut, liver, cardiac, brain and kidney functions; and vulnerability or susceptibility to health disorders in reliable manner. This reliable detection offers effective intervention leading to more cures and longer survival.

Moreover, the system replaces other expensive microbiome analysis with a highly sensitive novel metabolomic application. That is, as the system does not depend on the expensive next-generation sequencing for characterizing the gut microbiome, the prediction of the microbiome profile with this system is less expensive as compared to sequencing. That is, the system can potentially reduce the cost of determining the gut microbiome profile to 1/20th. Further, the system consumes less time to predict the microbiome than the next-generation sequencing technique-based prediction. That is, with the system, the turnaround time of the microbiome profile prediction is potentially reduced from 4-8 weeks to 24-48 hours. Further, as the blood sample collected on the dry blood card is used for predicting the microbiome profile, the process of collecting, transporting, and handling the sample (blood sample) is logistically easier with the system than the faecal sample-based prediction. In other words, the system eliminates the need of collecting faecal samples, in turn, avoids sample loss while predicting the microbiome profile.

Further, the system predicts the microbiome profile, thereby providing a glimpse into the potential applications and outcomes in predicting individual health. As the machine learning model of the present disclosure enables to identify the harmful and helpful microbiomes along with their relative abundances, the system offers a comprehensive snapshot of the microbial landscape within the individual. Moreover, as the system provides the phylum-wise distribution of predicted microbiomes, the system helps in understanding of the microbial composition at a broader taxonomic level. The prediction of the Firmicutes to Bacteroidetes (F/B) ratio with the system further contributes to the personalized characterization of the gut microbiome, a key parameter associated with metabolic health.

With the system, prediabetic, type 2 diabetes (T2D), overweight, obesity (Ob), stress associated anxiety and depression (SAD), vaginal microbial dysbiosis, and neurodegenerative diseases (NDD) are predicted effectively. As the metabolite markers are sensitive to the picomolar ($10^{-12}$ M, thousandth of a billionth) level, even a single marker for a disease could be potent enough to detect the onset of a disease with the system. In the system, the metabolome data is correlated with accuracy and specificity index with the microbiome profile that may be absent or present in a disease condition. Further, the system can serve not only as a predictive tool but also will lead to the development and design of potential probiotic intervention strategies at a community and individual level. This potential probiotic formulation for an individual could be a personalized probiotic supplement. In addition, this system can also provide information on other general health parameters (e.g., lipid profile, vitamin, and amino acid deficiencies, metabolic and immune status etc.) over and above the microbiome prediction. Also, the system is independent of disease area and can be useful for mental and physical wellness, enhancing sports performance, cosmetics testing and other such application areas where the microbiota can play a role. Therefore, the system can potentially replace many traditional pathological tests with a much higher significance, sensitivity, and low cost.

Moreover, by leveraging the metabolite-based microbiome prediction approach of the system as an add-on to existing mass spectrometry (MS) instruments, the applications of MS functionality are expanded across all makes of instruments. This integration empowers researchers to seamlessly incorporate microbiome profiling into their analyses, making MS applications universally adaptable.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation.

BRIEF DESCRIPTION OF THE DRAWINGS

The embodiments herein will be better understood from the following detailed description with reference to the drawings, in which:

FIG. 1 is a block diagram of a system for determining a microbiome profile of a host according to some embodiments herein;

FIG. 2 is an exploded view of a microbiome profile determination server of FIG. 1 according to some embodiments herein;

FIG. 3 is a flow diagram that illustrates a method of developing a machine learning model of FIG. 1 to determine a microbiome profile of a host according to some embodiments herein;

FIG. 4 is a flow diagram that illustrates a method of determining a microbiome profile of a host using the system of FIG. 1 according to some embodiments herein;

FIG. 5 illustrates an exemplary user interface (UI) of mass spectral data associated with a sample of a host according to some embodiments herein;

FIGS. 6A-6B are exemplary views that illustrate a network of predicted metabolite-microbiome pairs during the correlation analysis according to some embodiments herein;

FIG. 7 is a spider web plot that illustrates a correlation between metabolites and microbiomes according to some embodiments herein;

FIG. 8 is a graphical representation that illustrates a correlation between metabolites and abundance of microbiomes according to some embodiments herein;

FIG. 9 is a graphical representation that illustrates correlation strength between metabolites and microbes across different health conditions by regression according to some embodiments herein;

FIGS. 10A-10C are graphical representations that illustrate a percentage of predicted metabolite-microbiome pairs in different accuracy bins in different cohorts according to some embodiments herein;

FIGS. 11A-11C are graphical representations that show distribution of microbiome at a phylum level in different cohorts according to some embodiments herein;

FIGS. 12A-12C are graphical representations that show impact of microbiomes in different cohorts according to some embodiments herein;

FIGS. 13A-13C are graphical representations that show primary functions of microbiomes in different cohorts according to some embodiments herein;

FIG. 14 is a graphical representation that shows distribution of microbiome at a phylum level that is predicted with a machine learning model for a test patient, according to some embodiments herein;

FIG. 15 is a graphical representation that illustrates relative abundances of microbiome that is predicted with a machine learning model based on impact (harmful and helpful) for a test patient, according to some embodiments herein; and

FIG. 16 is a schematic diagram of a computer architecture in accordance with the embodiments herein.

DETAILED DESCRIPTION OF THE DRAWINGS

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

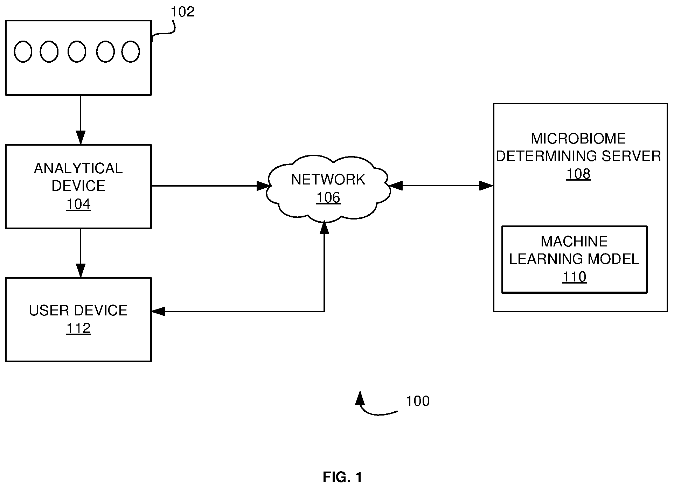

As mentioned, there is a need for determining gut microbiome at low cost in less time without any sample loss to detect early onset of a disease and/or develop a personalized probiotic-based solution. Embodiments herein provide a system and method for determining microbiome from host metabolome (blood metabolites) using a machine learning model. Referring now to the drawings, and more particularly to FIGS. 1 through 16, where similar reference characters denote corresponding features consistently throughout the figures, preferred embodiments are shown.

FIG. 1 is a block diagram of a system 100 for determining a microbiome profile of a host according to some embodiments herein. The system 100 includes a sample collection device 102, an analytical device 104, a network 106, a microbiome profile determination server 108 that includes a machine learning model 110, and a user device 112.

The microbiome profile determination server 108 includes a processor and a non-transitory computer-readable storage medium (or memory) storing one or more sequences of instructions, which when executed by the processor causes determination of the microbiome profile using the machine learning model 110. The microbiome profile determination server 108 may be a handheld device, a mobile phone, a kindle, a Personal Digital Assistant (PDA), a tablet, a music player, a computer, an electronic notebook or a Smartphone. The microbiome profile may be associated with gastrointestinal tract (gut), oral, vaginal, mucosa, or skin. In some embodiments, the microbiome profile is associated with the gut.

The sample collection device 102 is configured to collect a sample from the host. The host may include human, animal, and microbes. The sample may be a biological sample or body fluid. The sample may be at least one of whole blood, serum, plasma, faecal, saliva, skin tissues, microbial swabs from at least one of vaginal, oral or skin, or other body fluids including tears, sweat and urine. The sample collection device 102 may be at least one of Dry Blood Spot (DBS) card, blood collection tube, serum separator tube, ethylene diamine tetra acetic acid (EDTA) tube, stool collection container, saliva collection tubes, biopsy punches or the like.

In some embodiments, the sample collection device 102 is a Dry Blood Spot (DBS) card. The sample collection device 102 comprises of a small piece of filter paper that is attached to a plastic card. The sample may be collected from the host at one or more spots on the sample collection device 102. In some exemplary embodiments, the sample is collected on the sample collection device 102 by pricking the host's finger with a lancet, placing a small drop of blood sample onto the filter paper at five different spots, and allowing the blood to dry. Each spot may have 10-15 $\mu$L of blood sample. The sample collection device 102 may be sent for further analysis. The sample may be collected in other suitable sample collection devices, for example, blood collection tube.

The analytical device 104 is configured to analyse the sample that is collected on the sample collection device 102 and obtain spectral data of one or more metabolites present in the sample. Each spot in the sample collection device 102 may be punched and the sample in each spot is collected in a small tube containing 100 $\mu$L of a solvent mixture. A small circular hole punch may be used to remove a spot of sample from the sample collection device 102. The solvent mixture may include methanol and acetonitrile in 1:1 ratio. The samples in the small tubes are kept overnight in a refrigerator at 4∘C (degree Celsius). After overnight refrigeration, 50 $\mu$L of the sample in the small tubes is used for analysis with the analytical device 104. In some embodiments, the analytical device 104 is a liquid chromatography tandem mass spectrometry (LC-MS/MS) analyser. The analytical device 104 may be a Raman spectrometer, a nuclear magnetic resonance (NMR) spectrometer, a Fourier transform infrared (FTIR) spectrometer, a Fourier transform ion cyclotron resonance mass spectrometry (FT-ICR MS),or a capillary electrophoresis associated with detector.

In some embodiments, the analytical device 104 includes a separation unit, and a mass analyser. The separation unit separates different metabolites of the sample based on physical and chemical properties. The separation unit may be an ultra-performance liquid chromatography (UPLC). The mass analyser analyses the separated metabolites by measuring mass-to-charge ratio and outputs the mass spectral data of the one or more metabolites in the sample. The mass analyser may be an electrospray ionisation mass spectrometry (ESI-MS). The mass spectral data includes information about the mass-to-charge (m/z) ratios of the detected ions in the sample, relative abundance or intensity of the detected ions (area under the curve), and fragmentation pattern of ions. In some embodiments, the analytical device 104 is NMR spectrometer that analyses the sample and obtains NMR spectral data. In some embodiments, the analytical device 104 is Raman spectrometer that obtains Raman spectra of the sample.

The microbiome profile determination server 108 is configured to connect with the analytical device 104 and receive the spectral data associated with the sample from the analytical device 104 through the network 106. The network 106 is a wireless network or wired network. The network 106 is a combination of a wired network and a wireless network. In some embodiments, the network 106 is Local area network (LAN) or Internet. In some embodiments, a user may input the spectral data to the microbiome profile determination server 108 after obtaining the spectral data from the analytical device 104 through the user device 112. In some embodiments, the user device 112 is communicatively connected with the microbiome profile determination server 108 through the network 106 and includes a client-side program. The user device 112 may receive the spectral data from the analytical device 104 and input the spectral data to the microbiome profile determination server 108 using the client-side program. The client-side program may be a web application or a mobile application. The spectral data may be in at least one form of wave metrics Igor binary format (WIF), mass spectrometry extensible markup language (mzXML), mass spectrometry markup language (mzML), raw data file, or peak list file. In some embodiments, the spectral data is in a WIF format. The spectral data may be stored in a database.

The microbiome profile determination server 108 is further configured to generate metabolome data associated with the sample by analysing and annotating the spectral data of the sample against spectral data of known metabolites stored in a public database. The metabolome data may be generated by detecting peaks associated with the one or more metabolites in the sample, and matching values of the peaks with theoretical peak values of known metabolites in the public database. The peak values may include peak intensity, mass-to-charge (m/z), peak area, or full width at half maximum (FWHM) of peak. The public database may include kyoto encyclopedia of genes and genomes (KEGG), human metabolome database (HMDB), biochemical genetic and genomic (BiGG) knowledgebase, chemical entities of biological interest (ChEBI), food database (FoodDB), drug bank database, and other related databases. The metabolome data includes one or more metabolites in the sample with their mass to charge ratio (m/z) and relative abundance (concentrations). In some exemplary embodiments, the one or more metabolites includes (-)-Citramalic acid, (-)-Coralyne cation, (-)-Epigallocatechin, (-)-Epigallocatechin gallate, (-)-Epinephrine, (-)-Gallocatechin, (-)-Homoeriodictyol, (-)-Hydroxycitric acid lactone, (-)-Hydroxymatairesinol, (-)-Indolactam V, (-)-Medicarpin, (-)-N6-(2-Phenylisopropyl)adenosine, (-)-Neplanocin A, (-)-Nicotine, (-)-Nuciferine, (-)-O-Acetyl-D-mandelic acid, (-)-Quinic acid, (-)-Riboflavin, (-)Shikimic acid, (-)-Strychnine, (-)-Sulforaphene, (.+/-.)-2-Methylarachidonoyl-2'-fluoroethylamide, (.+/-.)-Cromakalim, (.+/-.)-Laurotetanine, (.+/-.)-Lefetamine, (.+/-.)-Lyoniresinol 2a-O-.beta.-D-glucopyranoside, (.beta.-D-Glucopyranosyloxy)(4-hydroxyphenyl)acetonitrile, (.epsilon.-phenylthiocarbamyl)lysine phenylthiohydantoin, ([1,2,4]Triazolo[3,4-b][1,3]benzothiazol-3-ylsulthio)acetic acid, ({4-Chloro-5-phenylthieno[2,3-d]pyrimidin-2-yl}methyl)dimethylamine, (+).alpha.-Tocopherol, (+)-.gamma.-Tocopherol.

The microbiome profile determination server 108 is configured to predict the microbiome profile of the host based on the metabolome data using the machine learning model 110. The microbiome profile includes phylum, genus and/or species level information on microbial population found in the faeces associated with the host with their relative abundance. The machine learning model 110 may be trained by mapping historical metabolites associated with the historical samples to the corresponding gut microbiome. The machine learning model 110 may be developed using one or more techniques of linear or non-linear regression including ridge, elastic net, random forest, and the like; and multivariate classification or grouping methodologies including principal component analysis (PCA) or principal coordinates analysis (PcoA), linear discriminant analysis (LDA), decision curve analysis (DCA) and clustering techniques such as k-means clustering, mean-shift clustering, density-based spatial clustering of applications with noise (DBSCAN), expectation-maximization (EM) clustering using gaussian mixture models (GMM), and agglomerative hierarchical clustering. The microbiome profile may be determined based on the strength of correlation between the metabolomic profile and the microbiome (metagenomic). The strength of correlation between the metabolomic and metagenomic profile provides a rank list of metagenome (microbiome) mapped on metabolites.

The microbiome profile determination server 108 is further configured to generate a microbiome report of the host based on the obtained microbiome profile. The microbiome report may include phylum, genus and/or species level distribution of microbiome, information on harmful and helpful microbiomes with the abundance, and a firmicutes to bacteriodetes (F/B) ratio. The generated microbiome report may be sent to the user device 112 through the client-side program. In some embodiments, the microbiome profile may be analysed manually to generate the microbiome report.

The determined microbiome profile and the microbiome report may be used to detect early disease onset and health disorders that are associated with gut microbes. Further, the microbiome profile and the microbiome report help in developing potential probiotic intervention strategies at a community and individual level (that is, a personalized probiotic supplement).

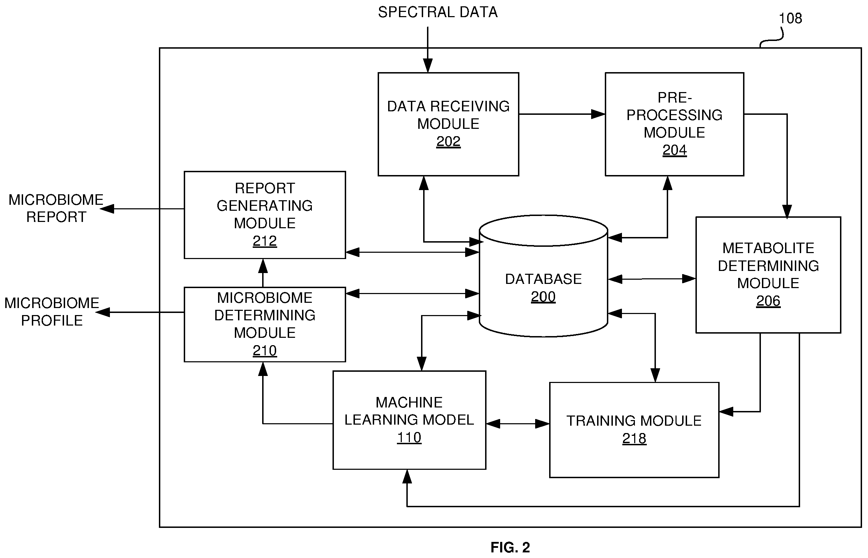

FIG. 2 is an exploded view of a microbiome profile determination server 108of FIG. 1 according to some embodiments herein. The microbiome profile determination server 108includes a database 200, a machine learning model 110, a data receiving module 202, a pre-processing module 204, a metabolite determining module 206, a training module 208, a microbiome determining module 210, and a report generating module 212.

The data receiving module 202 connects with the analytical device 104 or the user device 112 through the network 106 and receives spectral data of one or more metabolites in a sample taken from a host. In some embodiments, that spectral data is a mass spectral data that includes information about the mass-to-charge (m/z) ratios of the detected ions in the sample, relative abundance or intensity of the detected ions (area under the curve), and fragmentation pattern of ions. In some embodiments, the mass spectral data is in a WIF format. In some embodiments, the spectral data is NMR spectrum that encompasses details about the chemical shifts exhibited by nuclei in the sample and provides information on peak integration. The area under each peak in the spectrum provides information about the number of equivalent nuclei contributing to that peak. In some embodiments, the spectral data is Raman spectra which is a plot of intensity against the frequency shifts. Peaks in the spectrum represent vibrational modes of different molecular components in the sample.

The pre-processing module 204 pre-processes the spectral data by (i) converting the format of the spectral data from WIF format into mzXML format for downstream analysis; and (ii) removing background noise or artifacts that may interfere with the identification of metabolites from the mass spectral data. The pre-processing module 204 further converts the mzXML format of spectral data into axon binary format (ABF). The pre-processing module 204 may use one or more techniques known in the art for pre-processing the spectral data.

The metabolite determining module 206 identifies peaks associated with the one or more metabolites. The metabolite determining module 206 may select the peaks above a certain threshold of intensity or signal-to-noise ratio as relevant peaks corresponding to the one or more metabolites. The metabolite determining module 206 may use mass spectrometry data-independent analysis (MSDIAL) for peak detection. The positions of the peaks in the spectral data may be determined by the chemical shift, which is influenced by the local magnetic environment of the nuclei in different metabolites.

The metabolite determining module 206 further annotates the peaks associated with the one or more metabolites by matching values of the peaks with theoretical peak values of known metabolites in a public database to generate metabolome data. The metabolite determining module 206 may annotate the peaks by matching the observed m/z values with the theoretical m/z values of known metabolites in public databases (for example, mass Bank, national institute of standards and technology (NIST), the human metabolome database (HMDB)). From the annotation results, the metabolite determining module 206 generates the metabolome data of the sample (that is, one or more metabolites associated with the sample). In some embodiments, the metabolite determining module 206 may analyse the spectral data and calculate the area under each peak. The area under each peak in the spectrum is directly proportional to the number of nuclei contributing to that particular resonance signal. The metabolite determining module 206 may perform an integration process that involves summing up the signal intensities across the entire width of the peak. The resulting integrated area may provide a quantitative measure of the relative abundance of the corresponding metabolite or molecular species in the sample. In some embodiments, the metabolite determining module 206 may determine the metabolites and the concentration based on intensity of peaks in the spectral data. That is, the peak intensity is proportional to the concentration of the corresponding metabolites.

The training module 208 is configured to train the machine learning model 110 to predict a microbiome profile of the host from the metabolome data of the sample.

In some embodiments, historical blood samples are collected from historical hosts at one or more spots on the sample collection device 102 (dry blood spot card). The historical hosts may be associated with one or more diseases or disorders including diabetic (T2D), obese (Ob), stress associated anxiety and depression (SAD), neurodegenerative diseases (NDD, e.g., Parkinson's, Alzheimer, etc.), non-alcoholic fatty liver disease (NAFLD). Each spot in the sample collection device 102 is punched and the historical blood sample in each spot is collected in small tubes containing 100 μL of a solvent mixture (i.e. 1:1 ratio of methanol and acetonitrile). The historical blood samples in the small tubes are kept overnight in a refrigerator at $4^0$C (degree Celsius). After overnight refrigeration, 50 μL of historical blood samples from the small tubes are analysed using an analytical device 104 to obtain mass spectral data of the one or more metabolites in the historical blood samples. The metabolome data (one or more metabolites) associated with the historical blood samples is determined by analysing and annotating the spectral data of the historical blood samples against known metabolites in the public database.

In some embodiments, faecal samples (or poop) are collected from the historical hosts and scooped out from each end and the middle of the faecal samples in specimen containers. The specimen containers with the faecal samples are transferred immediately for further analysis. The faecal samples may be collected in DNA stabilizer containing glass beads to avoid the risk of DNA degradation. If the faecal samples are collected in the DNA stabilizer containing glass beads, vigorous shaking is required to mix the faecal sample properly with the DNA stabilizer. The collected faecal samples may be subsampled into aliquots for future application. The faecal samples may be frozen for long-term storage at -80°C. Before freezing, it is recorded for the consistency and texture of the poop according to Bristol Classification chart. The poop with hard and lump consistency may contain more microbial compositional variation.

After, DNA from the faecal samples is isolated within 24 hours to 48 hours of collection. The DNA may be isolated using DNEASY Power soil Kit (Qiagen) or QIAamp Fast DNA Fecal Mini Kit. In some embodiments, the DNA is eluted in nuclease-free water, rather in the buffer. The purity of the DNA is checked using nanodrop and qubit and the integrity is checked by agarose gel electrophoresis. The isolated DNA is stored at -80°C. The DNA may be subsampled into aliquots to avoid multiple freeze-thaw. The isolated DNA is subjected to 16S rDNA metagenomic sequencing (Next Generation Sequencing). The next generation data of the faecal samples is compared against known metagenomic databases to determine microbiome data in the faecal samples. The microbiome data includes phylum, and genus level information on microbial population found in the faecal samples. A list with various phyla and genera is made for each host data with relative abundance.

The training module 208 uses the metabolome data (one or more metabolites) associated with the historical blood samples and the microbiome data derived from the faecal samples of the historical hosts as training data to train the machine learning model 110. The training module 208 trains the machine learning model 110 by (i) performing, using a regression model, a correlation analysis between metabolome data associated with historical blood samples and microbiome data in historical faecal samples to obtain association patterns relating to historical metabolites and the corresponding microbiome based on high accuracy and high spearman correlation coefficient value; and (ii) training the machine learning model 110 by mapping the historical metabolites to the corresponding microbiome based on the association patterns, thereby acquiring the learned association patterns that enable the machine learning model 110 to predict the microbiome profile.

In some embodiments, the training module 208 uses a regression model to determine the strength of correlation between the metabolome data associated with the historical blood samples and the microbiome data derived from the faecal samples of the historical hosts. The training module 208 may perform comparative analysis of various regression methodologies to optimise the best correlated metabolite-microbiome pair based on false discovery rate (FDR), confidence level and correlation coefficient. The best regression model may be picked based on low FDR, high confidence level, and the like. The training module 208 may use Elastic Net regression model that predicts the microbiome abundance from the metabolome data. The training module 208 chooses microbiomes that meet or exceed a stringent accuracy threshold of 90%. This is, only the microbiomes whose predictions are highly accurate, with a confidence level of at least 90%, are considered. After selecting the microbiomes based on the accuracy threshold, the training module 208 may calculate the Spearman's rank correlation coefficient that measures the degree of association between the measured metabolite abundance values and the selected microbiome abundance values. Finally, the training module 208 selects metabolite-microbiome pairs based on the highest Spearman correlation coefficient values. The metabolite-microbiome pairs with a high accuracy and strong correlations between metabolites and microbiomes are considered as training data. Based on the correlation analysis, the training module 208 trains the machine learning model 110 with the training data. The machine learning model 110 may be learnt association patterns based on the selected metabolite-microbiome pairs and predict the microbiome profile based on the learnt association patterns. The machine learning model 110 may be a neural network model.

The microbiome determining module 210 predicts microbiome profile of the host from the metabolome data of the sample using the machine learning model 110 that is trained by the training module 208. The machine learning model 110 may extract one or more features of the metabolome data and correlate the one or more features with learned association patterns to predict the microbiome profile. The one or more features may include a presence of particular metabolite, concentration of metabolites, ratios of certain metabolites, temporal dynamics of metabolite concentrations, diversity indices of metabolites, and the like. The one or more features of the metabolome data may be extracted based on the relative abundance of the plurality of metabolites in the sample. The microbiome profile includes phylum, genus or species level information on microbial population associated with the host with the relative abundance.

The report generating module 212 generates a microbiome report of the host based on the microbiome profile. The microbiome report may include phylum, genus or species level distribution of microbiome, information on harmful and helpful microbiomes with the abundance, and a firmicutes to bacteriodetes (F/B) ratio. The report generating module 212 may perform taxonomic profiling, differential abundance analysis, functional profiling, machine learning based predictive modeling, or functional annotation to obtain the information on harmful and helpful microbiomes. The report generating module 212 may calculate the Firmicutes to Bacteroidetes ratio by dividing the abundance of Firmicutes by the abundance of Bacteroidetes. The report generating module 212 further sends the generated microbiome report to the user device 112 through the client-side program.

The training module 208 is further configured to retrain the machine learning model 110 by mapping the metabolome data associated with the sample to the microbiome profile of the host that is predicted, thereby refining the predictive capabilities of the machine learning model 110 and enhancing the accuracy of the machine learning model 110 over successive iterations.

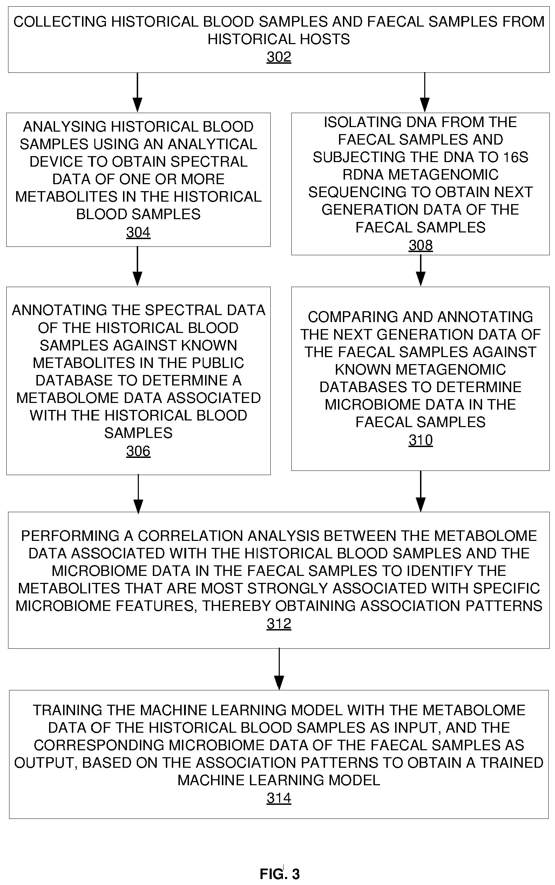

FIG. 3 is a flow diagram that illustrates a method of developing a machine learning model 110of FIG. 1 to determine a microbiome profile of a host according to some embodiments herein. At step 302, historical blood samples and faecal samples are collected from historical hosts. At step 304, historical blood samples are analysed using an analytical device 104 to obtain spectral data of one or more metabolites in the historical blood samples. At step 306, the spectral data of the historical blood samples is annotated against known metabolites in the public database to determine a metabolome data (one or more metabolites) associated with the historical blood samples. At step 308, DNA from the faecal samples is isolated and subjected to 16S rDNA metagenomic sequencing to obtain next generation data of the faecal samples. At step 310, the next generation data of the faecal samples is compared and annotated against known metagenomic databases to determine microbiome data in the faecal samples.

At step 312, a correlation analysis is performed between the metabolome data associated with the historical blood samples and the microbiome data in the faecal samples to identify the metabolites that are most strongly associated with specific microbiome features, thereby obtaining association patterns (or a rank list of microbiome mapped on metabolites). A regression model may be used to perform the correlation analysis. The association patterns are obtained based on high accuracy and high spearman correlation coefficient value. At step 314, the machine learning model 110 is trained with the metabolome data of the historical blood samples as input, and the corresponding microbiome data of the faecal samples as output, based on the association patterns to obtain a trained machine learning model 110. The machine learning model 110 may be a neural network model. The learned association patterns enable the trained machine learning model 110 to predict a microbiome profile of the host from a metabolome data associated with the host in real-time.

In some exemplary embodiments, the raw metabolome batch files from the analytical device 104 are separated into samples of control, obesity, and type 2 diabetes (T2D). The samples are counted, for example, control = 23, obesity=32, T2D= 33. The samples of control are combined into one single file and similar files are made for rest two categories. Then, the metabolites and their associated abundance values are organized in a row-wise manner after removing duplicates. After that, mean, standard deviation (SD), number of occurrences in the overall sample (n), confidence interval (CI), coefficient of variation (CV), and other statistical measures such as log transformation and normalization are calculated. The common and unique metabolites between all three categories are find out and the metabolites are filtered on the basis of a 95% confidence interval, between 20% SD and 1.5 CV. The statistical significance is found by applying post-hoc stats (ANOVA).On the basis of significance and abundance, the metabolites are grouped into the classes of high, medium, and low.

Similarly, the raw data from 16S rDNA metagenomic sequencing are separated into samples of control, obesity, and T2D and counted as control=15, obesity=08, T2D=15. Then, phylum, genus, and species levels are organized and mean, standard deviation, number of occurrences in the overall sample (n), and coefficient of variation (CV) are calculated. The significance between the three categories is found on the basis of CV and by applying post-hoc stats.

Significant metabolites and significant genus information are taken and the correlation between them is found. Then, they are regressed to find out the strength of the correlation.

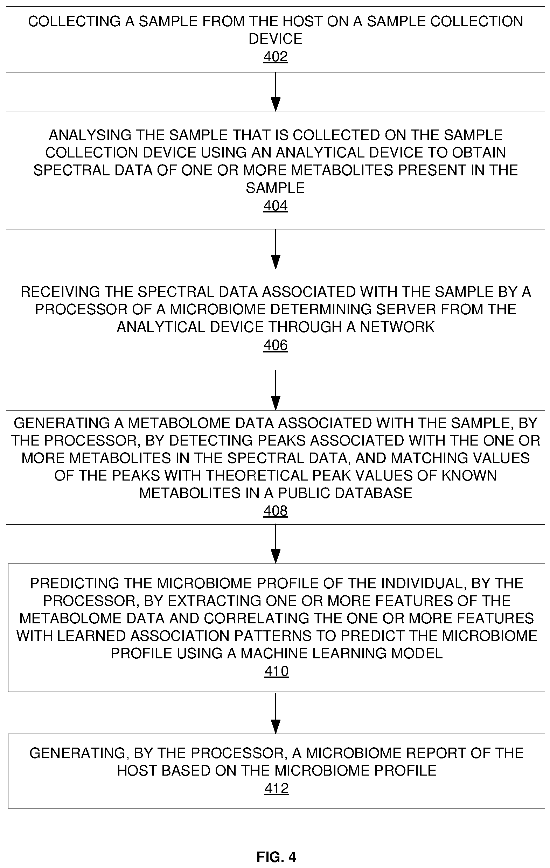

FIG. 4 is a flow diagram that illustrates a method of determining a microbiome profile of a host using the system 100 of FIG. 1 according to some embodiments herein. At step 402, a sample from the host is collected on a sample collection device 102. The sample may be at least one of whole blood, serum, plasma, faeces, saliva, skin tissues, microbial swabs from at least one of vaginal, oral or skin, or other body fluids including tears, sweat and urine. In some embodiments, the sample collection device 102 is a Dry Blood Spot (DBS) card. At step 404, the sample that is collected on the sample collection device 102 is analysed using an analytical device 104 to obtain spectral data of one or more metabolites present in the sample. In some embodiments, the analytical device 104 is a liquid chromatography tandem mass spectrometry (LC-MS/MS) analyser that obtains mass spectral data of the one or more metabolites in the sample. The mass spectral data includes information about the mass-to-charge (m/z) ratios of the detected ions in the sample, relative abundance or intensity of the detected ions (area under the curve), and fragmentation pattern of ions.

At step 406, the spectral data associated with the sample is received by a processor of a microbiome profile determination server 108 from the analytical device 104 through a network 106. At step 408, metabolome data associated with the sample is generated, by the processor, by detecting peaks associated with the one or more metabolites in the sample, and matching values of the peaks with theoretical peak values of known metabolites in a public database. The peaks values may include at least one of peak intensity, mass-to-charge (m/z), peak area, or full width at half maximum (FWHM) of peak. The metabolome data includes one or more metabolites in the sample with relative abundance. At step 410, the microbiome profile of the host is predicted by the processor by extracting one or more features of the metabolome data and correlating the one or more features with learned association patterns to predict the microbiome profile using a machine learning model 110.

At step 412, a microbiome report of the host is generated, by the processor, based on the microbiome profile. The microbiome report includes phylum, genus or species level distribution of microbiome, information on harmful and helpful microbiomes with the abundance, and a firmicutes to bacteriodetes (F/B) ratio.

In some embodiments, the one or more features of the metabolome data are extracted based on the relative abundance of throne or more metabolites in the sample. The one or more features of the metabolome data include a presence of particular metabolite, a concentration of metabolites, ratios of certain metabolites, temporal dynamics of metabolite concentrations, and diversity indices of metabolites.

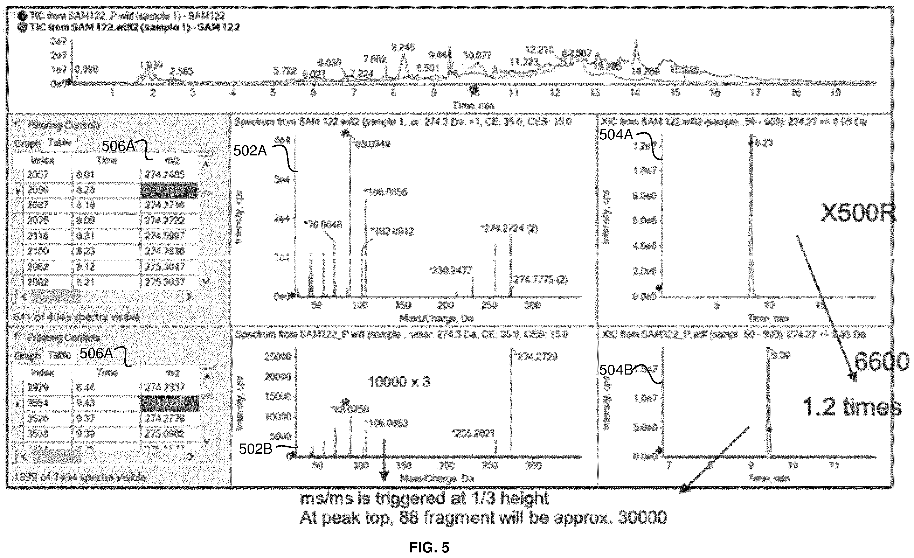

FIG. 5 illustrates an exemplary user interface (UI) of mass spectral data associated with a sample of a host according to some embodiments herein. The UI includes spectral plots 502A, and 502B that display the mass spectrum. In the spectral plots 502A-B, the intensity is plotted in Y-axis and mass-to-charge ratio (m/z) or mass is plotted against X-axis. Each peak in the spectral plots 502A-B represents an ion with a specific mass-to-charge ratio. The UI further includes plots 504A, and 504B that that represent the intensity of ions detected by the mass spectrometer (an analytical device 104) over a period of time. In the plots 504A-B, the intensity is plotted in Y-axis and time is plotted in X-axis. The intensity of each ion is a measure of the abundance or concentration of that particular ion in the sample at a specific point in time. The UI further includes data tables 506A, and 506B that provide additional information about each detected peak. The data tables 506A-B may facilitate data analysis and comparison of multiple peaks.

In some exemplary embodiments, a study related to predicting a microbiome profile from human metabolome was performed with type 2 diabetes (T2D), and obesity patients. The study was approved by the Institutional Ethics Committee of AIIMS, Bhubaneswar (Ref. No. T/ EM-F/ Endocri/ 21/ 75) and NISER (IEC No: NISER/ IEC/ 2023-01). T2D, and obesity patients were recruited through AIIMS and control patients were recruited through NISER following the inclusion and exclusion criteria mentioned in Table 1.

Table 1: Inclusion and exclusion criteria

| Inclusion criteria | |
| --- | --- |
| Age | 18-59 years |
| Gender | Male and Female (Both) |
| BMI | $\geq 25$ (obesity) |
| Blood glucose | $\geq 126$ mg/dL (7 mmol/L) (T2D) |
| HbAlc | $\geq 6.5\%$ (T2D) |
| Exclusion criteria | |
| Alcohol consumption | $\geq 20$ grams/ day |
| Other types of diabetes | T1DM, GDM |

(continued)

| Exclusion criteria | |
| --- | --- |
| Antibiotics intake | Less than 3 weeks |

Study participants were separated into three cohorts such as control, obesity, and type 2 diabetes (T2D) and were counted, for example, control = 23, obesity=30, T2D= 34. The clinical data of the study participants were given in Table 2.

Table 2: Clinical data of the study participants

| Characteristics | Control | T2D | Obesity |
| --- | --- | --- | --- |
| Total | 23 | 30 | 34 |
| Gender (numbers) | | | |
| Male | 13 | 20 | 13 |
| Female | 10 | 10 | 21 |
| Age (years, mean $\pm$ SD) | 27.09 $\pm$ 10.41 | 50.20 $\pm$ 13.18 | 35.91 $\pm$ 12.12 |
| Weight (kg, mean $\pm$ SD) | 63.24 $\pm$ 10.13 | 66.63 $\pm$ 9.67 | 97.16 $\pm$ 21.88 |
| Height (cm, mean $\pm$ SD) | 164.6 $\pm$ 9.75 | 160.5 $\pm$ 10.40 | 158.9 $\pm$ 9.84 |
| BMI (mean $\pm$ SD) | 23.24 $\pm$ 2.61 | 25.94 $\pm$ 3.24 | 38.45 $\pm$ 7.61 |
| Fasting blood glucose (mg/ dl) (mean $\pm$ SD) | 89.87 $\pm$ 9.07 | 138.8 $\pm$ 41.05 | 107.5 $\pm$ 41.04 |
| HbA1c (%) (mean $\pm$ SD) | NA | 8.36 $\pm$ 1.86 | 7.01 $\pm$ 2.03 |

Faecal samples were collected from the participants belonging to each cohort in a sterile 50 ml conical tube with scoop attached. The faecal samples were transported to the lab and kept at -80 °C until extracted for DNA. Genomic DNA from the stool was extracted using a QIAamp Fast DNA Stool Mini Kit (Qiagen, Germany) as described in the manufacturers' protocol. Quantification and assessment for quality of the extracted DNA was performed using a Nanodrop instrument and Qubit 4.0 fluorometer. Next, sequencing targeting the V3- V4 region was accomplished using 520F and 802R primer pair and Ion 530 chip kit on Ion Gene Studio S5 System (Thermo Fisher Scientific, MA, USA). Raw FASTQ data generated after sequencing were processed using a designated 16S analysis pipeline in the Ion ReporterTM software to get the Operational Taxonomic Unit (OUT) abundance table.

The peripheral blood sample was collected from the healthy (control) and diseased (T2D and obesity) individuals by easy DIY finger-pricking method using a sterile lancet and spotted (5 drops) on the Whatman paper of DBS card. After collecting the samples, the cards were air-dried, placed in a desiccant-containing bag and shipped to the Mass spec facility for metabolite extraction. Next, a 3 mm disk was punched from the blood-spotted DBS card, 5 disks of each individual were pooled and transferred to a well of 24 well plates containing the extraction solvent (ice-cold 1:1- methanol: acetonitrile with 0.1% formic acid and internal standard CPA). Gentle shaking was employed to facilitate extraction of the metabolites for 2 hours and kept the supernatant for additional 2 hours at 4°C for precipitation. Then, the samples were centrifuged for 15 minutes at 16,000g at 4°C and collected the supernatant in a fresh 1.5 ml microcentrifuge tube. The solvent is evaporated using a SpeedVac Vacuum Concentrator with medium speed setting for 1.5 hour and resuspended the pellet in 50 $\mu$l of acetonitrile- H2O (1:1; v/v) solution by vortexing. Finally, the samples were centrifuged for 15 minutes at full speed, collected the supernatant into the LC-MS vials, and proceeded with the LC-MS acquisition.

An AB Sciex TripleTOF® 6600 mass spectrometer and an AB Sciex Ultrahigh performance liquid chromatography (UHPLC) system integrated with a quaternary AD pump, autosampler, degassing system, controller, and column oven were used to carry out the untargeted LC-MS/MS metabolomics. A 5 $\mu$l volume sample was injected into the column using an autosampler that was adjusted to 4°C. To separate the samples using reverse phase liquid chromatography (RPLC), a kinetex 2.6 m C18 column (100 $\times$ 4.6 mm) was utilised. The column oven temperature was set to30°C, and the system was operated at a flow rate of 0.5 ml per minute. MiliQ water with 0.1% formic acid (mobile phase A) and acetonitrile with 0.1% formic acid (mobile phase B) were the mobile phases used for gradient separation. Each run was followed by a methanol (blank) wash and lasted a total of 20 minutes. The MS equipment was calibrated for positive and negative modes with two calibrant solutions prior to running the samples (APCI positive and negative calibration solution, Sciex). The analytes were ionised using a DuoSpray ion source, an electron spray ionization (ESI) device that worked in both positive and negative modes with the Analyst® TF software, after LC elution. The mass range for both parent and product ion scans was adjusted at 50-900 m/z in high-sensitivity mode, and PeakView software was used to process the data. All the solvents and reagents used for the LC/MS run were of MS grade (J.T.Baker®, Phillipsburg, NJ, USA).

The untargeted metabolomics data is then processed using the open-source MS-DIAL (ver. 4.90) software pipeline for peak picking, peak alignment, peak identification, and quantification following data-dependent acquisition (DDA). Soft ionisation (ionisation type), chromatography (separation type), conventional or data-dependent MS/ MS (MS technique type), profile data for both MS1 and MS/ MS (data type), specific ion modes, and metabolomics were the parameter settings that were used for the study in the software (target omics). Metabolite identification was performed using the integrated MassBank-NIST (National Institute of Standards and Technology, Maryland, USA) library after extracting the retention time (Rt) and accurate mass (m/z) information of the parent ions. Finally, the relative peak intensities of the identified metabolites were calculated from the area under the curve for each identified peak and used for further downstream analyses.

The first step in the downstream analyses was to predict microbiome abundance values from metabolite abundance data. Primarily, both the metabolite and microbiome abundance values were standardized using the mean and standard deviation, and then used ElasticNet to predict abundance values. ElasticNet automatically selects the most relevant features, and the relevant features were bolstered with 5-fold cross-validation to ensure the model's resilience and generalizability. The ElasticNet regression model ran for each of the three cohorts presented in Table 2. It should be noted that for the regression model, only patients from 21 Control, patients from 27 T2D, and 28 patients from Obesity groups were used. This is because both metabolite and microbiome abundance values were available only for these patients in this study. To evaluate the accuracy of the predictions, 95% confidence intervals were employed for microbiome. If a predicted abundance value for a microbiome fell within the 95% confidence interval range, it was assigned a value of 1. Otherwise, a value of 0 was assigned. Model predictions were translated into binary labels and this approach enabled to compute true positive, true negative, false positive, and false negative values, facilitating the calculation of accuracy using the following formula (1):

$$Accuracy = \frac{(True\ positive + True\ negative)}{(True\ positive + True\ negative + False\ positive + False\ negative)}$$

After implementing the regression model, a corresponding microbiome was computed for each measured metabolite within the patient cohorts. Selection criteria involved choosing microbiome that met or exceeded a stringent accuracy threshold of 90%. Then the Spearman's rank correlation coefficient was calculated between the measured metabolite abundance values and microbiome abundance values using the following formula (2).

$$\rho = 1 - [(6 * \Sigma d^2) / (n * (n^2 - 1))]$$

where $\rho$ is the Spearman's rank correlation coefficient, $\Sigma d^2$ is the sum of squared differences between the ranks and n is the number of paired observations.

The metabolite-microbiome pairs were selected based on the highest Spearman correlation coefficient values. This yielded unique pairs with a remarkably high accuracy and strong correlations between metabolites and microbiome and can be used for training the machine learning model 110 to predict the microbiome profile.

In some exemplary embodiments, Table 3.1 illustrates the training data associated with the metabolites that are used for training the machine learning model 110.

Table 3.2: Training data _ Metabolites

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S97_SAM 17 | 0 | 0 | 0 | 6182.2 18 | 0 | 0 | 0 | 0 | 0 | 0 |
| S96_SAM 01 | 0 | 0 | 0 | 0 | 0 | 0 | 0 | 0 | 0 | 0 |
| S99_SAM 101 | 11894. 27 | 0 | 10667. 54 | 0 | 4137. 368 | 7898.176 | 8342.365 | 57218 .44 | 0 | 5353. 866 |
| S100_SA M15 | 0 | 0 | 0 | 13261. 18 | 0 | 0 | 0 | 0 | 0 | 0 |
| S101_SA M56 | 0 | 60457 .71 | 0 | 7528.1 97 | 0 | 0 | 0 | 0 | 0 | 0 |
| S102_SA M49 | 0 | 0 | 0 | 0 | 0 | 0 | 0 | 0 | 0 | 0 |
| S107_SA M103 | 6979.1 21 | 0 | 23121. 93 | 0 | 4533. 712 | 10446.04 | 10710.05 | 50554 .92 | 8605. 555 | 0 |
| S36_SAM 57 | 0 | 51984 .19 | 0 | 10675. 77 | 0 | 0 | 0 | 0 | 0 | 0 |
| S42_SAM 59 | 0 | 26271 .77 | 0 | 0 | 0 | 0 | 0 | 0 | 0 | 0 |
| S47_SAM 54 | 0 | 0 | 0 | 0 | 0 | 0 | 0 | 0 | 0 | 0 |
| S39_SAM 14 | 0 | 15430 0.8 | 0 | 0 | 0 | 0 | 0 | 0 | 0 | 0 |

| Metabolite | ((2,6-dichlorophenyl)sulfonyl)methionine | ((propane-2,2-diylbis(4,1-phenylene))bis(oxy))bis(3,6,9,12-tetraoxatetradecane-14,1-diyl)bis(2-methylacrylate) | (1,1-dioxido-3-oxo-1,2-benzisothiazol-2(3h)--yl)acetic acid | (1,2,9,10-tetramethoxy-6-methyl-5,6,6a,7-tetrahydro-4h-dibenzo[de,g]quinolin-3-yl)methanol | (1-(4-bromophenyl)cyclopropyl)methanamine | (1-{[(tert-butoxycarbonyl)amino]methyl}cyclohexyl)acetic acid | (1.xi.)-1,5-anhydro-1-(5,7-dihydroxy-2-(4-hydroxyphenyl)-4-oxo-4h-chromen-8-yl)-6-o-((2e)-6-hydroxy-2,6-dimethylocta-2,7-dienoyl)-d-glucitol | (11e,15z)-9,10,13-trihydroxyoctadeca-11,15-dienoic acid | (16e)-3-hydroxy-16-(phenylmethylene)estra-1,3,5(-10)-trien-17-one | (17z)-16-(acetyloxy)-6-hydroxy-4,8,14-trimethyl-3,7-dioxo-18-norcholesta-17,24-dien-21-oic acid |
|---|---|---|---|---|---|---|---|---|---|---|
| S41_SAM 53 | 0 | 0 | 0 | 0 | 0 | 0 | 0 | 0 | 0 | 0 |
| S45_SAM 18 | 0 | 0 | 0 | 0 | 0 | 0 | 0 | 0 | 0 | 0 |
| S52_SAM 19 | 0 | 7076.935 | 0 | 11709.8 | 0 | 0 | 0 | 0 | 0 | 0 |
| S40_SAM 51 | 0 | 0 | 0 | 0 | 0 | 0 | 0 | 0 | 0 | 0 |
| S37_SAM 05 | 0 | 11307.18 | 0 | 6887.78 | 0 | 0 | 0 | 0 | 0 | 0 |
| S53_SAM 60 | 0 | 51833.65 | 0 | 18513.28 | 0 | 0 | 0 | 0 | 0 | 0 |
| S48_SAM 16 | 0 | 0 | 0 | 9106.3 | 0 | 0 | 0 | 0 | 0 | 0 |
| S38_SAM 50 | 0 | 0 | 0 | 5834.798 | 0 | 0 | 0 | 0 | 0 | 0 |
| S56_SAM 58 | 0 | 0 | 0 | 11083.28 | 0 | 0 | 0 | 0 | 0 | 0 |
| S57_SAM 102 | 11309.66 | 0 | 0 | 0 | 2936.206 | 9944.399 | 7741.605 | 21943.2 | 22877.95 | 27755.04 |
| S68_SAM 83 | 0 | 0 | 5020.044 | 0 | 0 | 5845.637 | 5192.471 | 89509.38 | 18801.25 | 16104.26 |

EP 4 471 790 B1

14

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S63_SAM 115 | 17096. 1 | 0 | 0 | 0 | 0 | 0 | 0 | 48242 .9 | 0 | 12345 .83 |
| S69_SAM 78 | 12830. 65 | 0 | 17663. 78 | 0 | 0 | 0 | 3902.461 | 20568 9.1 | 25314 .25 | 0 |
| S73_SAM 75 | 0 | 0 | 9374.3 49 | 0 | 0 | 3953.962 | 0 | 41962 .82 | 41245 .18 | 9321. 304 |
| S74_SAM 108 | 8549.6 02 | 0 | 0 | 0 | 0 | 0 | 0 | 36755 .18 | 0 | 12907 .55 |
| S76_SAM 121 | 0 | 0 | 0 | 0 | 0 | 0 | 0 | 0 | 0 | 6172. 373 |
| S78_SAM 94 | 6650.1 72 | 0 | 0 | 0 | 0 | 0 | 5377.559 | 15246 .6 | 0 | 0 |
| S80_SAM 71 | 13267. 77 | 0 | 5684.3 49 | 0 | 3188. 059 | 0 | 4697.502 | 53771 .07 | 22418 .5 | 0 |
| S87_SAM 82 | 6399.029 | 0 | 12637.12 | 0 | 0 | 0 | 5192.471 | 40739.67 | 0 | 16104.26 |
| S89_SAM 68 | 0 | 0 | 10317. 01 | 0 | 5980.456 | 0 | 7538.907 | 67478 .94 | 0 | 0 |
| S90_SAM 76 | 13091. 89 | 0 | 6639.8 85 | 0 | 3976. 674 | 0 | 0 | 58647 .58 | 0 | 0 |
| S92_SAM 114 | 0 | 0 | 0 | 0 | 0 | 0 | 0 | 0 | 0 | 16836 .27 |

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S93_SAM 127 | 2841.2 11 | 0 | 0 | 0 | 0 | 0 | 0 | 29265 3.5 | 0 | 0 |
| S94_SAM 98 | 12636. 82 | 0 | 11502. 54 | 0 | 0 | 0 | 5673.257 | 39775 .77 | 0 | 0 |
| S106_SA M72 | 7746.4 33 | 0 | 0 | 0 | 0 | 0 | 5284.083 | 63961 .25 | 37816 .63 | 8333. 539 |
| S108_SA M42 | 0 | 2139. 574 | 0 | 13723. 27 | 0 | 0 | 0 | 0 | 0 | 0 |
| S109_SA M81 | 9646.4 35 | 0 | 4547.2 71 | 0 | 0 | 0 | 5192.471 | 21745 9.9 | 23856 .79 | 11746 .72 |
| S110_SA M48 | 0 | 0 | 0 | 23398. 06 | 0 | 0 | 0 | 0 | 0 | 0 |
| S111_SA M88 | 7406.6 73 | 0 | 0 | 0 | 8153. 427 | 0 | 4754.953 | 48733 .19 | 0 | 7087. 26 |
| S112_SA M93 | 0 | 0 | 0 | 0 | 3135. 081 | 0 | 0 | 0 | 0 | 0 |
| S49_SAM 13 | 0 | 0 | 0 | 0 | 0 | 0 | 0 | 0 | 0 | 0 |
| S29_SAM 39 | 0 | 0 | 0 | 31799. 45 | 0 | 0 | 0 | 0 | 0 | 0 |
| S31_SAM 45 | 0 | 18128 .7 | 0 | 20203. 97 | 0 | 0 | 0 | 0 | 0 | 0 |

EP 4 471 790 B1

(continued)

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S35_SAM 62 | 0 | 0 | 7710.0 92 | 0 | 0 | 0 | 8748.768 | 48978 .88 | 0 | 0 |
| S34_SAM 66 | 0 | 0 | 0 | 0 | 0 | 0 | 6106.001 | 10152 2.6 | 15460 .64 | 0 |
| S59_SAM 64 | 12997. 77 | 0 | 0 | 0 | 0 | 0 | 5642.707 | 58537 .01 | 0 | 6741. 369 |
| S25_SAM 47 | 0 | 42681 .82 | 0 | 10407. 3 | 0 | 0 | 0 | 0 | 0 | 0 |
| S23_SAM 46 | 0 | 66128 .23 | 0 | 24536. 41 | 0 | 0 | 0 | 0 | 0 | 0 |
| S71_SAM 79 | 11983. 33 | 0 | 0 | 0 | 0 | 7176.99 | 5570.609 | 13005 4.6 | 18521 .13 | 10716 .58 |
| S64_SAM 84 | 9107.9 51 | 0 | 8708.4 65 | 0 | 0 | 0 | 7251.205 | 33410 .32 | 29052 .93 | 0 |
| S72_SAM 85 | 9963.8 92 | 0 | 7347.7 92 | 0 | 3483. 476 | 13189.84 | 5908.266 | 50570 .67 | 0 | 0 |
| S75_SAM 120 | 0 | 0 | 0 | 0 | 0 | 0 | 0 | 0 | 0 | 0 |
| S77_SAM 122 | 3860.9 08 | 0 | 0 | 0 | 5481. 197 | 4456.933 | 0 | 8743. 518 | 0 | 0 |
| S79_SAM 80 | 0 | 0 | 0 | 0 | 0 | 0 | 5307.781 | 33712 .58 | 0 | 26620 .34 |

(continued)

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S81_SAM 95 | 5629.7 57 | 0 | 0 | 0 | 8440. 641 | 8305.944 | 7135.42 | 41421 .34 | 7946. 477 | 7921. 392 |
| S88_SAM 90 | 0 | 0 | 10317. 01 | 0 | 5980.456 | 0 | 7538.907 | 67478 .94 | 0 | 0 |
| S91_SAM 124 | 5174.1 9 | 0 | 10075. 01 | 0 | 6469. 752 | 7553.045 | 0 | 19499 2.1 | 0 | 0 |
| S103_SA M110 | 6521.2 6 | 0 | 0 | 0 | 0 | 0 | 0 | 33203 .37 | 0 | 0 |
| S104_SA M99 | 0 | 0 | 16747. 88 | 0 | 0 | 0 | 6704.92 | 86470 .5 | 0 | 0 |
| S105_SA M105 | 8813.9 54 | 0 | 19665. 79 | 0 | 8346. 519 | 19244.8 | 5239.665 | 36911 .91 | 0 | 4179. 554 |
| S24_SAM 32 | 0 | 17279 .94 | 0 | 21787. 41 | 0 | 0 | 0 | 0 | 0 | 0 |
| S28_SAM 27 | 0 | 0 | 0 | 0 | 0 | 0 | 0 | 0 | 0 | 0 |
| S05_SAM 22 | 0 | 20860 .86 | 0 | 20771. 13 | 0 | 0 | 0 | 0 | 0 | 0 |
| S06_SAM 23 | 0 | 0 | 0 | 7053.8 83 | 0 | 0 | 0 | 0 | 0 | 0 |
| S01_SAM 10 | 0 | 0 | 0 | 10542. 65 | 0 | 0 | 0 | 0 | 0 | 0 |

18

| Metabolite | ((2,6-dichlor opheny l) sulfonyl) me thionin e | ((prop ane-2,2-diylbis(4,1-pheny lene)) bis(ox y)) bis( 3,6,9, 12-tetrao xatetr adeca ne-14,1-diyl) bis(2-methy lacryl ate) | (1,1-dioxido -3-oxo-1,2-benziso thiazol-2(3h)--yl)aceti c acid | (1,2,9,1 0-tetrame thoxy-6-methyl -5,6,6a, 7-tetrahy dro-4h-dibenz o [de,g] quinoli n-3-yl)met hanol | (1-(4-brom ophe nyl)c yclop ropyl )met hana mine | (1-{[(tert-butoxycar bonyl) ami no]methyl} cyclohex yl) acetic acid | (1.xi.)-1,5-anhydro-1-(5,7-dihydrox y-2-(4-hydroxyp henyl)-4-oxo-4h-chromen-8-yl)-6-o-((2e)-6-hydroxy-2,6-dimethyl octa-2,7-dienoyl)-d-glucitol | (11e,1 5z)-9,10,1 3-trihyd roxyo ctadec a-11,15-dienoi c acid | (16e)-3-hydro xy-16-(phen ylmet hylene ) estra-1,3,5(-10)-trien-17-one | (17z)-16-(acety loxy)-6-hydro xy-4,8,14 -trimet hyl-3,7-dioxo-18-norch olesta-17,24-dien-21-oic acid |
|---|---|---|---|---|---|---|---|---|---|---|
| S09_SAM 25 | 0 | 0 | 0 | 13617. 9 | 0 | 0 | 0 | 0 | 0 | 0 |
| S08_SAM 24 | 0 | 0 | 0 | 10494.84 | 0 | 0 | 0 | 0 | 0 | 0 |
| S26_SAM 28 | 0 | 19320 .06 | 0 | 8344.7 12 | 0 | 0 | 0 | 0 | 0 | 0 |
| S02_SAM 11 | 0 | 6642. 132 | 0 | 5902.3 09 | 0 | 0 | 0 | 0 | 0 | 0 |
| S21_SAM 41 | 0 | 0 | 0 | 12355. 07 | 0 | 0 | 0 | 0 | 0 | 0 |
| S19_SAM 34 | 0 | 0 | 0 | 55290. 13 | 0 | 0 | 0 | 0 | 0 | 0 |
| S18_SAM 44 | 0 | 16028 .28 | 0 | 7969.3 05 | 0 | 0 | 0 | 0 | 0 | 0 |
| S22_SAM 40 | 0 | 0 | 0 | 10778. 77 | 0 | 0 | 0 | 0 | 0 | 0 |
| S30_SAM 43 | 0 | 0 | 0 | 16064. 44 | 0 | 0 | 0 | 0 | 0 | 0 |
| S20_SAM 30 | 0 | 30505 .37 | 0 | 11255. 05 | 0 | 0 | 0 | 0 | 0 | 0 |

EP 4 471 790 B1

In some exemplary embodiments, Table 3.2 illustrates the training data associated with the microbiome that are used for training the machine learning model 110.

Table 3.2: Training data _ Microbes

| Genus | Acetan aerobac terium | Acid amin ococ cus | Acinet obacte r | Actin obaci llus | Aero monas | Aggreg atibacte r | Akker mansia | Alicyc liphilu s | Alistip es | Allison ella |
|---|---|---|---|---|---|---|---|---|---|---|
| S97_SAM17 | 0 | 0.01 | 0 | 0.04 | 0 | 0 | 0 | 0 | 0.04 | 0.1 |
| S96_SAM01 | 0 | 4.78 | 0 | 0 | 0 | 0 | 0 | 0 | 5.08 | 0.03 |
| S99_SAM101 | 0 | 0 | 0 | 0 | 0 | 0 | 0 | 0 | 0 | 0.09 |
| S100_SAM15 | 0.01 | 0 | 0 | 0 | 0 | 0.01 | 0 | 0 | 0.23 | 0 |
| S101_SAM56 | 0 | 0 | 0 | 0 | 0 | 0 | 0 | 0 | 0 | 0.09 |
| S102_SAM49 | 0 | 0 | 0.02 | 0 | 0 | 0 | 0 | 0 | 0.01 | 0.02 |
| S107_SAM103 | 0 | 0.04 | 0 | 0 | 0 | 0 | 0 | 0 | 0.18 | 0 |
| S36_SAM57 | | 0 | | 0 | | | 0 | | 7.5986 72 | 0 |
| S42_SAM59 | | 0 | | 0 | | | 0 | | 1.0206 75 | 0.9945 04 |
| S47_SAM54 | | 0 | | 0 | | | 0.1222 | | 3.1771 89 | 0.1629 33 |
| S39_SAM14 | | 0 | | 0 | | | 0 | | 4.1072 2 | 0 |
| S41_SAM53 | | 0 | | 0 | | | 0 | | 0.0697 47 | 0 |
| S45_SAM18 | | 0.079 114 | | 0 | | | 0 | | 8.9926 16 | 0 |
| S52_SAM19 | | 0 | | 0 | | | 0 | | 0 | 0 |
| S40_SAM51 | | 0 | | 0 | | | 0 | | 1.0277 49 | 1.1305 24 |
| S37_SAM05 | | 0 | | 0 | | | 0.1997 67 | | 0.2663 56 | 0 |
| S53_SAM60 | | 0 | | 0 | | | 0 | | 0 | 0 |
| S48_SAM16 | | 0 | | 0 | | | 0 | | 0.3997 09 | 0 |
| S38_SAM50 | | 0 | | 0 | | | 0 | | 0.7214 02 | 0.0343 52 |
| S56_SAM58 | | 0.091 617 | | 0 | | | 0 | | 0 | 0.0229 04 |
| S57_SAM102 | | 0 | | 0.027 886 | | | 0.9620 75 | | 4.5036 25 | 0 |
| S68_SAM83_x | 0 | 0.48 | 0.01 | 0 | 0 | 0 | 0 | 0 | 0 | 0.15 |
| S63_SAM115_x | 0 | 0 | 0 | 0 | 0 | 0 | 0 | 0 | 0 | 0.04 |
| S69_SAM78_x | 0 | 0.03 | 0 | 0 | 0 | 0 | 0 | 0 | 0 | 0.14 |
| S73_SAM75_x | 0 | 0 | 0 | 0 | 0 | 0 | 0.09 | 0 | 2.8 | 0 |

| Genus | Acetan aerobac terium | Acid amin ococ cus | Acinet obacte r | Actin obaci llus | Aero monas | Aggreg atibacte r | Akker mansia | Alicyc liphilu s | Alistip es | Allison ella |
|---|---|---|---|---|---|---|---|---|---|---|
| S74_SAM108_ x | 0 | 0 | 0.03 | 0 | 0 | 0 | 0 | 0 | 0 | 0.05 |
| S76_SAM121_ x | 0 | 0 | 0 | 0 | 0 | 0 | 0.02 | 0 | 0.92 | 0.02 |
| S78_SAM94_x | | 0 | 0 | | | | 0 | | 0.1243 2 | 0 |
| S80_SAM71_x | | 0 | 0.144 404 | | | | 0 | | 0.6016 85 | 0.0722 02 |
| S87_SAM82_x | | 0 | 0 | | | | 0 | | 0.0519 21 | 0 |
| S89_SAM68_x | | 0 | 0 | | | | 0 | | 0 | 0 |
| S90_SAM76_x | | 0 | 0 | | | | 0 | | 2.4204 9 | 0.1407 26 |
| S92_SAM114_ x | | 0.273 66 | 0 | | | | 0 | | 0.0684 15 | 0.3420 75 |
| S93_SAM127_ x | | 0 | 0 | | | | 0.2161 38 | | 0.3602 31 | 0 |
| S94_SAM98_x | | 0 | 0 | | | | 0 | | 0 | 0 |
| S106_SAM72_ x | 0 | 0.03 | 0 | 0 | 0 | 0 | 0 | 0 | 0 | 0.03 |
| S108_SAM42_ x | 0 | 0 | 0 | 0 | 0 | 0 | 0 | 0 | 7.23 | 0 |
| S109_SAM81_ x | 0 | 0.15 | 0 | 0 | 0 | 0 | 0.01 | 0 | 0.24 | 0.78 |
| S110_SAM48_ x | 0 | 0 | 0 | 0 | 0 | 0 | 0 | 0.01 | 0.9 | 0.05 |
| S111_SAM88_ x | 0 | 0 | 0.04 | 0 | 0 | 0 | 0 | 0.01 | 0.93 | 0.04 |
| S112_SAM93_ x | 0 | 0 | 0 | 0 | 0 | 0 | 0 | 0 | 0 | 0.11 |
| S49_SAM13_ x | 0 | 0 | 0 | 0 | 0 | 0 | 0 | 0 | 0.01 | 0.06 |
| S29_SAM39_ x | 0 | 0 | 0 | 0 | 7.39 | 0 | 0 | 0 | 0.08 | 0 |
| S31_SAM45_ x | 0 | 0.02 | 0.01 | 0 | 0 | 0 | 0 | 0 | 0 | 0.15 |
| S35_SAM62_ x | 0 | 0 | 0.02 | 0 | 0 | 0 | 0 | 0 | 0 | 0.05 |
| S34_SAM66_ x | 0 | 0 | 0 | 0 | 0 | 0 | 0 | 0 | 0 | 0.03 |
| S59_SAM64_ x | 0 | 0.12 | 0 | 0 | 0 | 0 | 0 | 0 | 0 | 0.17 |
| S25_SAM47_ x | 0 | 0 | 0.05 | 0 | 0.02 | 0 | 0 | 0 | 1.36 | 0.04 |
| S23_SAM46_ x | 0 | 1.26 | 0 | 0 | 0 | 0 | 0 | 0 | 4.7 | 0 |
| S68_SAM83_y | 0 | 0.48 | 0.01 | 0 | 0 | 0 | 0 | 0 | 0 | 0.15 |

EP 4 471 790 B1

| Genus | Acetan aerobac terium | Acid amin ococ cus | Acinet obacte r | Actin obaci llus | Aero monas | Aggreg atibacte r | Akker mansia | Alicyc liphilu s | Alistip es | Allison ella |
|---|---|---|---|---|---|---|---|---|---|---|
| S63_SAM115_ y | 0 | 0 | 0 | 0 | 0 | 0 | 0 | 0 | 0 | 0.04 |
| S69_SAM78_y | 0 | 0.03 | 0 | 0 | 0 | 0 | 0 | 0 | 0 | 0.14 |
| S73_ SAM75_y | 0 | 0 | 0 | 0 | 0 | 0 | 0.09 | 0 | 2.8 | 0 |
| S74_SAM108_ y | 0 | 0 | 0.03 | 0 | 0 | 0 | 0 | 0 | 0 | 0.05 |
| S76_SAM121_ y | 0 | 0 | 0 | 0 | 0 | 0 | 0.02 | 0 | 0.92 | 0.02 |
| S78_SAM94_y | | 0 | 0 | | | | 0 | | 0.1243 2 | 0 |
| S80_SAM71_y | | 0 | 0.144 404 | | | | 0 | | 0.6016 85 | 0.0722 02 |
| S87_SAM82_y | | 0 | 0 | | | | 0 | | 0.0519 21 | 0 |
| S89_SAM68_y | | 0 | 0 | | | | 0 | | 0 | 0 |
| S90_SAM76_y | | 0 | 0 | | | | 0 | | 2.4204 9 | 0.1407 26 |
| S92_SAM114_ y | | 0.273 66 | 0 | | | | 0 | | 0.0684 15 | 0.3420 75 |
| S93_SAM127_ y | | 0 | 0 | | | | 0.2161 38 | | 0.3602 31 | 0 |
| S94_SAM98_y | | 0 | 0 | | | | 0 | | 0 | 0 |
| S106_SAM72_ y | 0 | 0.03 | 0 | 0 | 0 | 0 | 0 | 0 | 0 | 0.03 |
| S108_SAM42_ y | 0 | 0 | 0 | 0 | 0 | 0 | 0 | 0 | 7.23 | 0 |
| S109_SAM81_ y | 0 | 0.15 | 0 | 0 | 0 | 0 | 0.01 | 0 | 0.24 | 0.78 |
| S110_SAM48_ y | 0 | 0 | 0 | 0 | 0 | 0 | 0 | 0.01 | 0.9 | 0.05 |
| S111_SAM88_ y | 0 | 0 | 0.04 | 0 | 0 | 0 | 0 | 0.01 | 0.93 | 0.04 |
| S112_SAM93_ y | 0 | 0 | 0 | 0 | 0 | 0 | 0 | 0 | 0 | 0.11 |
| S49_SAM13_y | 0 | 0 | 0 | 0 | 0 | 0 | 0 | 0 | 0.01 | 0.06 |
| S29_SAM39_y | 0 | 0 | 0 | 0 | 7.39 | 0 | 0 | 0 | 0.08 | 0 |
| S31_SAM45_y | 0 | 0.02 | 0.01 | 0 | 0 | 0 | 0 | 0 | 0 | 0.15 |
| S35_SAM62_y | 0 | 0 | 0.02 | 0 | 0 | 0 | 0 | 0 | 0 | 0.05 |
| S34_SAM66_y | 0 | 0 | 0 | 0 | 0 | 0 | 0 | 0 | 0 | 0.03 |
| S59_SAM64_y | 0 | 0.12 | 0 | 0 | 0 | 0 | 0 | 0 | 0 | 0.17 |

EP 4 471 790 B1

| Genus | Acetan aerobac terium | Acid amin ococ cus | Acinet obacte r | Actin obaci llus | Aero monas | Aggreg atibacte r | Akker mansia | Alicyc liphilu s | Alistip es | Allison ella |
|---|---|---|---|---|---|---|---|---|---|---|
| S25_SAM47_y | 0 | 0 | 0.05 | 0 | 0.02 | 0 | 0 | 0 | 1.36 | 0.04 |
| S23_SAM46_y | 0 | 1.26 | 0 | 0 | 0 | 0 | 0 | 0 | 4.7 | 0 |

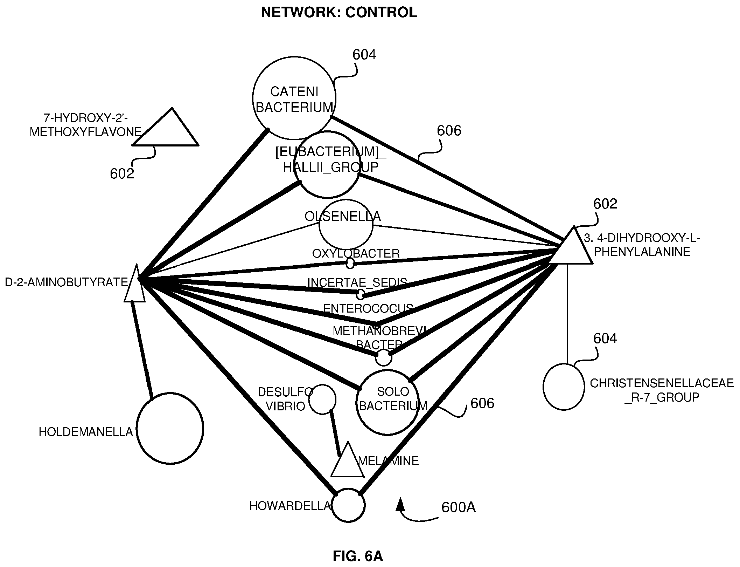

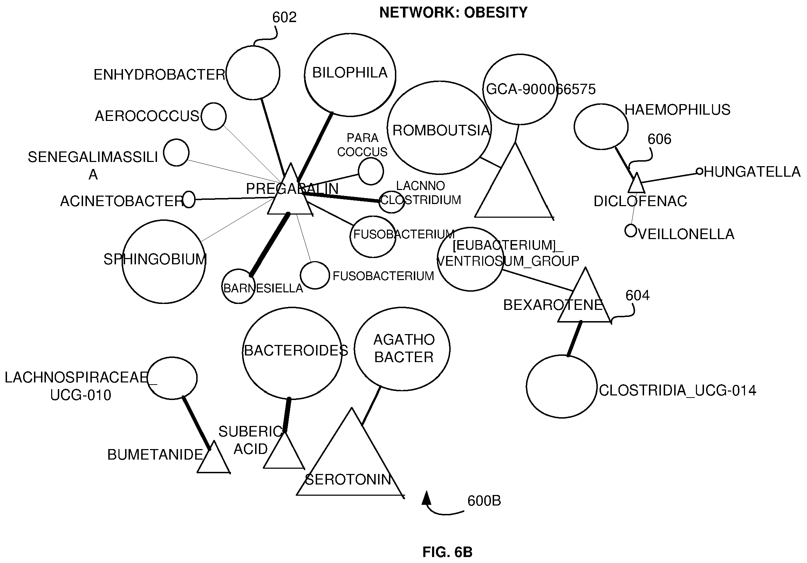

FIGS. 6A-6B are exemplary views that illustrate a network of predicted metabolite-microbiome pairs during the correlation analysis according to some embodiments herein. In some embodiments, the representation of metabolites and genera are shown as the network. The nodes indicate metabolite and genera and the edges represent the strength of the relation between the metabolite and the genera. In FIG. 6A, the network 600A represents the predicted metabolite-microbiome pairs for the cohort: control (that is, healthy individuals). In FIG. 6B, the network 600B represents the predicted metabolite-microbiome pairs for the cohort: obesity (that is, obese individuals). The networks 600A and 600B include triangles 602 showing the metabolites, and circles 604 showing the microbiome with edges 606. The width of the edges 606 indicates strength of connection between the metabolites (triangles 602) and the microbes (circles 604). Size of the circles 604 and triangles 602 indicates abundance or concentration of microbes and metabolites, respectively.

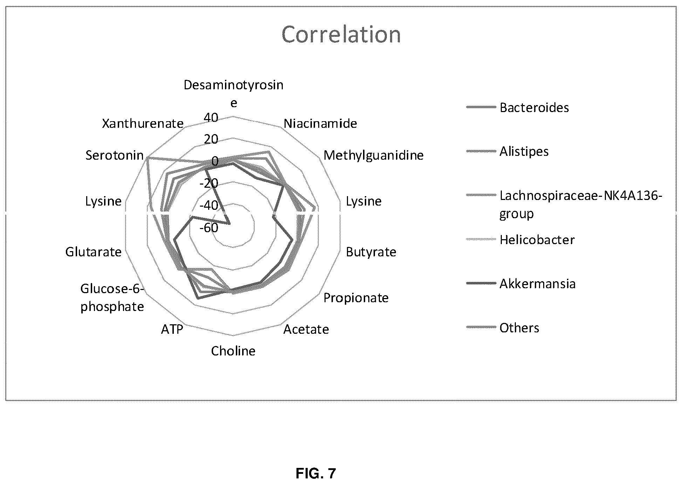

FIG. 7 is a spider web plot that illustrates a correlation between metabolites and microbiomes according to some embodiments herein. In FIG. 7, the metabolites are depicted on the periphery and the bacterial genera are depicted in the centre of the spider web plot. Lines extend from each bacterial genus towards the metabolites on the periphery, indicating the correlation strength between the metabolite and the genus. The length of the lines is proportional to the strength of the correlation. In FIG.7, the color-coded lines and their lengths help in quickly identifying which genera are strongly correlated with particular metabolites. The correlation is stronger when a bacterial group is closer to a metabolite on the outer edge (or periphery).

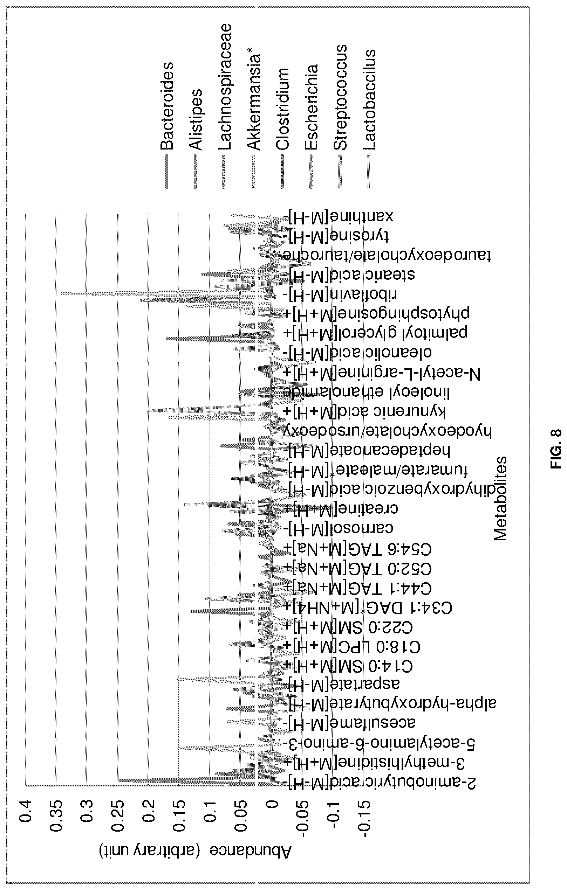

FIG. 8 is a graphical representation that illustrates a correlation between metabolites and abundance of microbiomes according to some embodiments herein. In the graphical representation, the metabolites are plotted against the X-axis and a bacterial abundance or concentration is plotted against the Y-axis with each bacterial strain distinguished by different colors. The graphical representation shows how each metabolite is connected to several bacteria with different concentrations. This data is valuable for discerning the strength of a metabolite's correlation with different bacterial ranks. Additionally, this data aids in evaluating instances where a metabolite is associated with more than one bacterium, allowing to identify which correlations are stronger and which are weaker.

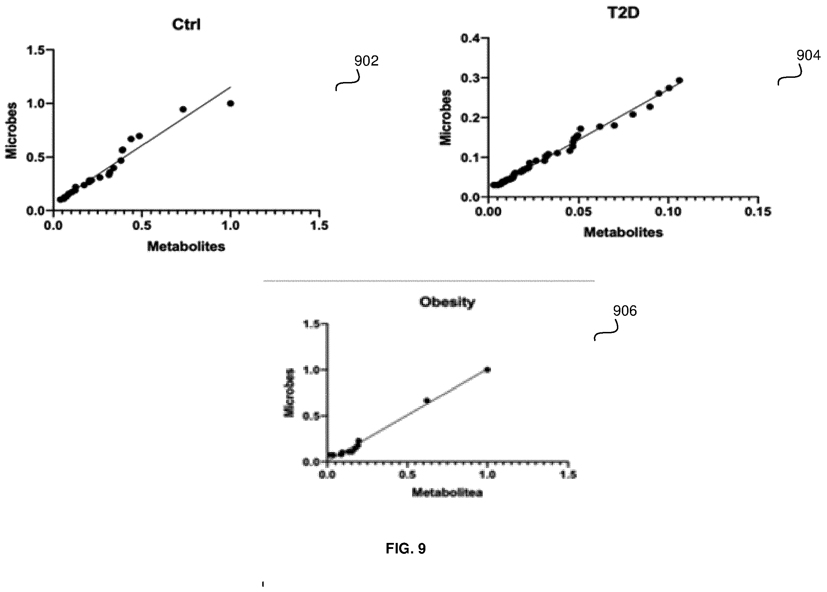

FIG. 9 is a graphical representation that illustrates correlation strength between metabolites and microbes across different health conditions by regression according to some embodiments herein. The graphical representation 902 represents the strength of correlation between metabolites and microbes for healthy individuals (control), the graphical representation 904 represents the strength of correlation between metabolites and microbes for type 2 diabetes (T2D), and the graphical representation 906 represents the strength of correlation between metabolites and microbes for obesity. In the graphical representations 902-906, metabolites are plotted against X-axis, and the microbes are plotted against Y-axis. The graphical representations 902-906 enable to visually assess the strength of correlation between metabolites and microbes for each health condition. Strong correlations may be indicated by tightly clustered points.

Further, statistical measures such as 95% confidence intervals (CI) are considered to quantify the uncertainty around the correlation estimates. Additionally, measures of goodness of fit, such as R-squared values, may be employed to assess how well the regression model fits the data. The statistical measures associated with the regression analysis across different health conditions are given in Table 4.

Table 4

| | Microbes associated with control | Microbes associated with T2D | Microbes associated with obesity |
|---|---|---|---|
| **Best fit values** | | | |
| Y Intercept | 0.06411 | 0.01800 | 0.003761 |
| Slope | 1.088 | 2.527 | 1.005 |
| **95% CI (profile likelihood)** | | | |
| Y Intercept | 0.02986 to 0.09836 | 0.01588 to 0.02011 | -0.02494 to 0.03246 |
| Slope | 0.9857 to 1.190 | 2.467 to 2.587 | 0.9250 to 1.084 |
| **Goodness of Fit** | | | |
| Degrees of freedom | 25 | 67 | 10 |
| R squared | 0.9507 | 0.9905 | 0.9875 |
| Sum of squares | 0.08232 | 0.002530 | 0.01164 |
| Sy.x | 0.05738 | 0.006145 | 0.03412 |
| **Number of points** | | | |
| # of X values analyzed | 69 | | 12 |

(continued)

| Number of points | | | |
|---|---|---|---|
| # of Y values analyzed | 69 | | 12 |

In some exemplary embodiments, the machine learning model 110 was trained by a method as shown in FIG. 3 based on the selected metabolite-microbiome pairs. Each of the three cohorts (control, T2D and Obesity) were split into an 80-20 ratio and 80% of the patients in a cohort was used to train the machine learning model 110 and the remaining 20% was used for testing purposes. As ground-truth microbiome abundance values were available for the test patients in the dataset, accuracy was calculated by the formula (1) and results have been presented in Table 5.

Table 5

| | Control | T2D | Obesity |
|---|---|---|---|
| Total number of Metabolite-microbiomes pairs predicted by model with accuracy > 90% | 884569 | 1048575 | 27604241 |
| Number of unique Metabolite-microbiomes pairs with accuracy > 90% | 177 | 168 | 160 |

In Table 5, first row reports the total number of metabolite-microbiome pairs predicted by the regression model with an accuracy greater than 90%, and second row reports the unique metabolite-microbiome pairs that have an accuracy greater than 90% and have the highest Spearman's rank correlation coefficient.

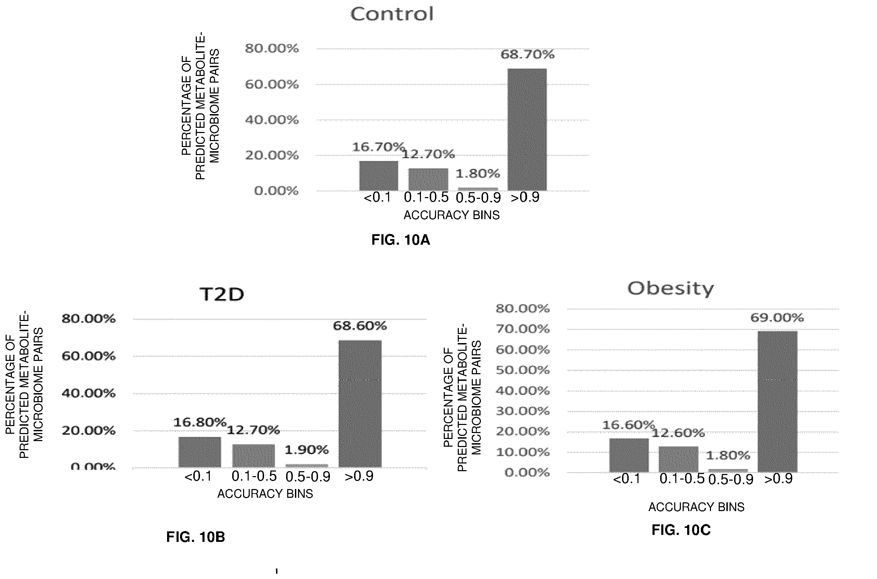

FIGS. 10A-10C are graphical representations that illustrate a percentage of predicted metabolite-microbiome pairs in different accuracy bins in different cohorts according to some embodiments herein. In some exemplary embodiments, accuracy associated with the different cohorts such as control, T2D, and obesity is categorized into one or more accuracy bins such as <0.1, 0.1-0.5, 0.5-0.9, and >0.9. The distribution of accuracy and the corresponding count of predicted metabolite-microbiome pairs for each accuracy bin are illustrated in the graphical representations. FIG. 10A represents the count of predicted metabolite-microbiome pairs for each accuracy bin in control cohort; FIG. 10B represents the count of predicted metabolite-microbiome pairs for each accuracy bin in T2D cohort; and FIG. 10C represents the count of predicted metabolite-microbiome pairs for each accuracy bin in obesity cohort. The number of predicted metabolite-microbiome pairs in each accuracy bin have been represented as percentages. In the graphical representations as shown in FIGS. 10A-10C, the one or more accuracy bins are plotted against X-axis, and the percentage of predicted metabolite-microbiome pairs is plotted against Y-axis. As shown in FIGS. 10A-B, the vast majority of predicted metabolite-microbiome pairs have an accuracy greater than 0.9 (i.e., 90%) for all the cohorts, such as the control, T2D, and obesity cohorts.

In the training dataset including 180 microbiomes, a comprehensive analysis is conducted to identify key attributes for 92 microbiomes, including their associated genus names, phyla, impact (harmful or helpful), and primary functions.

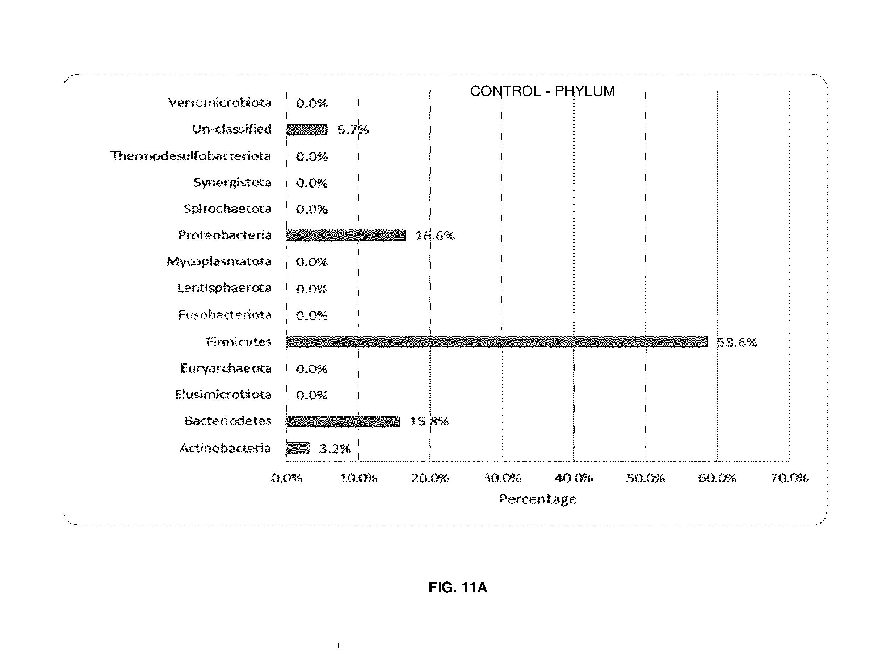

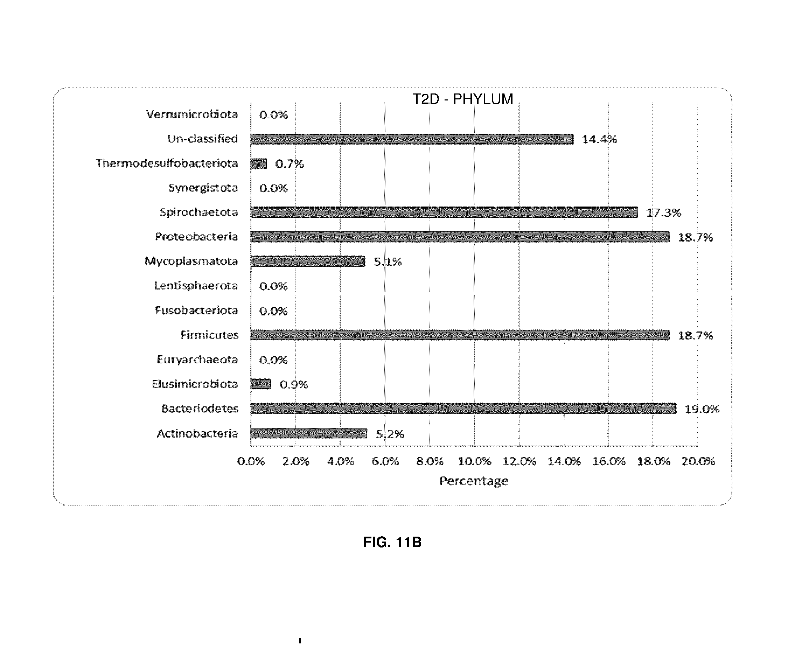

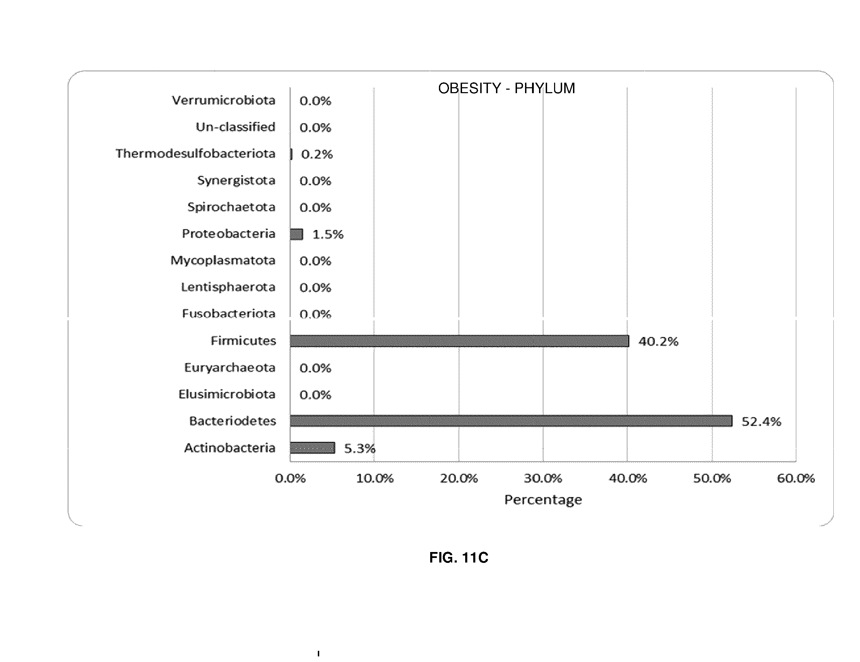

FIGS. 11A-11C are graphical representations that show distribution of microbiome at a phylum level in different cohorts according to some embodiments herein. FIG. 11A depicts the distribution of the microbiomes (phyla) in control (healthy) cohort. FIG. 11B depicts the distribution of the microbiomes in T2D cohort; and FIG. 11C depicts the distribution of the microbiomes in obesity cohort.

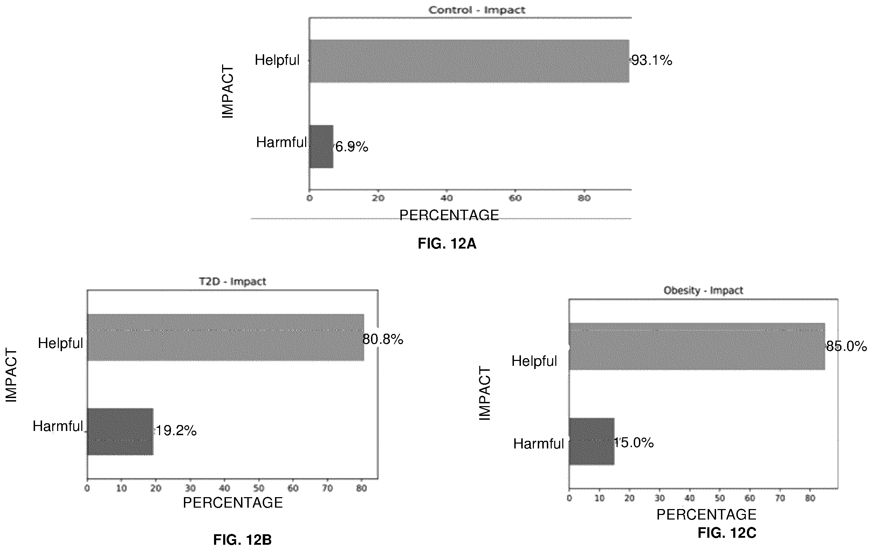

FIGS. 12A-12C are graphical representations that show impact of microbiomes in different cohorts according to some embodiments herein. FIG. 12A depicts the impact (harmful or helpful) of the microbiomes in control (healthy) cohort; FIG. 12B depicts the impact of the microbiomes in T2D cohort; and FIG. 12C depicts the impact of the microbiomes in obesity cohort.

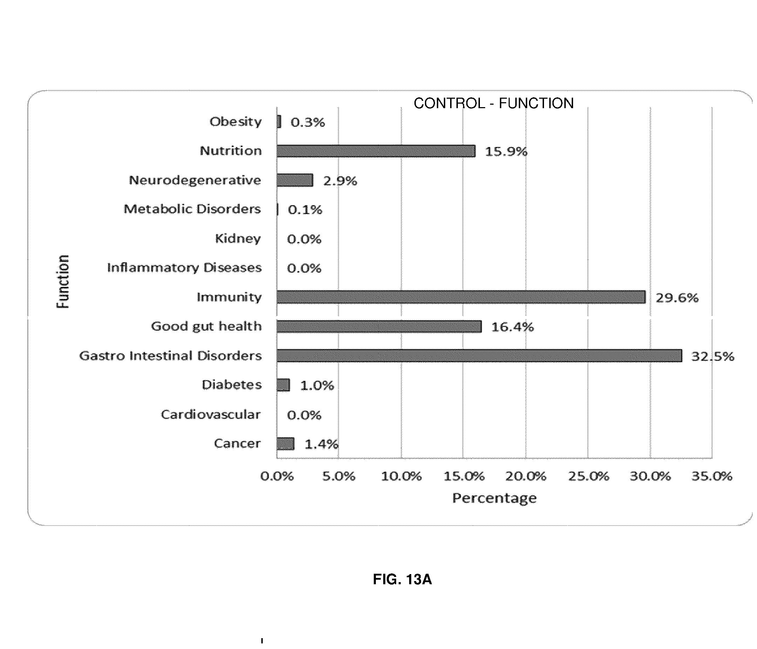

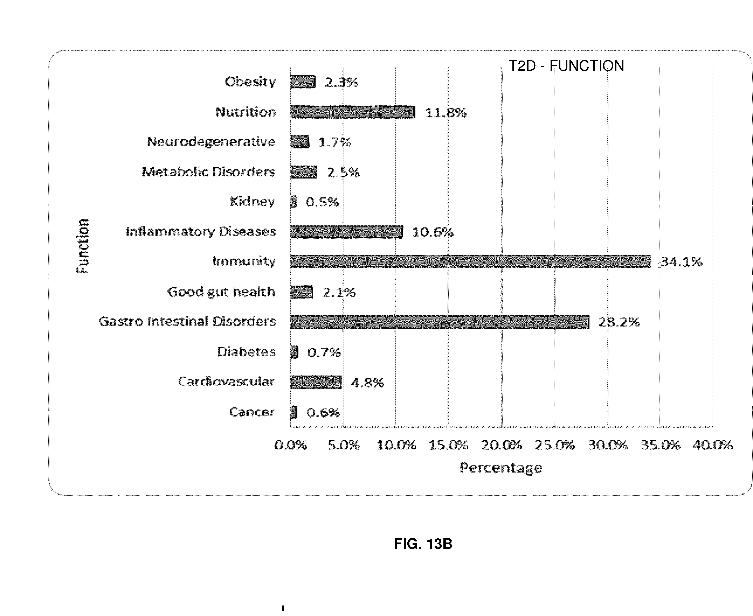

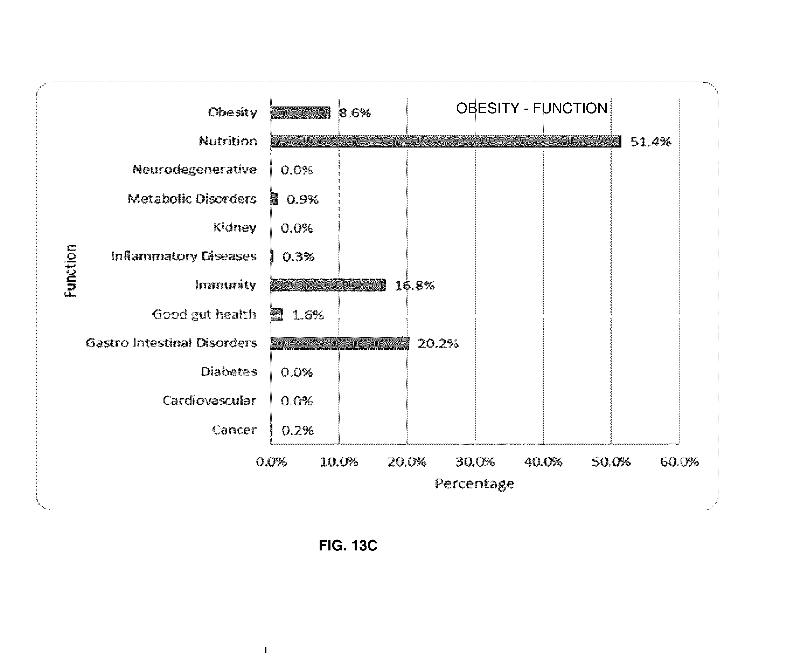

FIGS. 13A-13C are graphical representations that show primary functions of microbiomes in different cohorts according to some embodiments herein. FIG. 13A depicts the primary function of the microbiomes in control (healthy) cohort; FIG. 13B depicts the primary function of the microbiomes in T2D cohort; and FIG. 13C depicts the primary function of the microbiomes in obesity cohort.

Based on analysis of FIGS. 11A-13C, Acidaminococcus, classified under Firmicutes, is identified as harmful with an impact on immunity, capable of activating serious immune-related adverse events and leading to inflammatory responses and metabolic diseases. On the other hand, Agathobacter, also classified under Firmicutes, is recognized as helpful, specifically influencing immunity by being a significant producer of Butyrate-a short-chain fatty acid known for its anti-inflammatory properties. Similarly, Akkermansia from the Verrucomicrobia phylum is considered helpful in managing diabetes by controlling blood glucose levels and safeguarding against insulin resistance. Alistipes, falling under the Bacteroidetes phylum, is noted for its helpful role in mitigating various inflammatory diseases, including liver fibrosis, colorectal cancer, colitis, cardiovascular disease, and mood disorders.

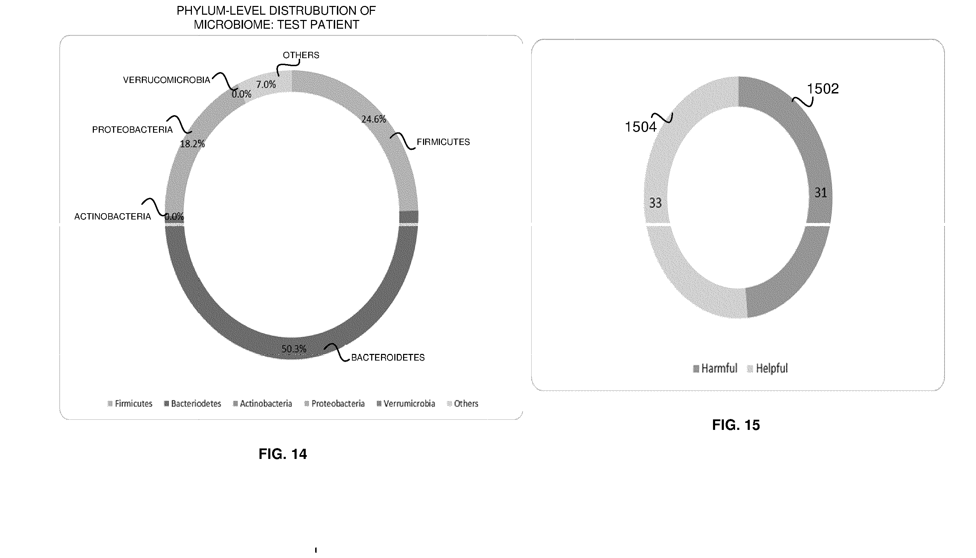

FIG. 14 is a graphical representation that shows distribution of microbiome at a phylum level that is predicted with a machine learning model 110 for a test patient, according to some embodiments herein. The test patient (37 year old male with a body-mass index of 30.1) whom microbiomes are not measured using NGS data is used for the analysis, and microbiomes and the abundance values are predicted with the machine learning model 110 using a method as shown in FIG. 4. The results on the distribution of the predicted microbiomes at the phylum level for the test patient is shown in FIG. 14. From the graphical representation, 50.3% of Bacteroidetes phylum, 24.6% of Firmicutes phylum 18.2% of proteobacteria phylum and 7% of other phyla are identified for the test patient.

FIG. 15 is a graphical representation that illustrates relative abundances of microbiome that is predicted with a machine learning model 110 based on impact (harmful and helpful) for a test patient, according to some embodiments herein. In the graphical representation, a pie section 1502 represents the harmful microbiomes and a pie section 1504 represents the beneficial microbiomes. For the test patient, a firmicutes to bacteriodetes (F/B) ratio of 1.3 is computed. From the graphical representation, the actual number of harmful microbiome present in the test patient is 31 and the actual number of beneficial microbiome present in the test patient is 33. Even though the number of harmful and beneficial microbiomes are approximately the same, their relative abundances vary, and this test patient has a higher percentage of abundance values expressed by harmful microbiomes according to F/B ratio.

The results obtained from the machine learning model 110, particularly when applied to a novel test patient without ground-truth measurements, provide a glimpse into the potential applications and outcomes in predicting individual health. The identification of harmful and helpful microbiomes, along with their relative abundances, offers a comprehensive snapshot of the microbial landscape within the individual. Moreover, the phylum-wise distribution of predicted microbiomes enriches the understanding of the microbial composition at a broader taxonomic level. The prediction of the Firmicutes to Bacteroidetes (F/B) ratio further contributes to the personalized characterization of the gut microbiome, a key parameter associated with metabolic health. These findings not only shed light on the potential for precision microbiome profiling but also open avenues for personalized health interventions. The ability to discern specific microbial functionalities and their abundances lays the groundwork for targeted probiotic interventions, allowing for the modulation of the microbiome towards a more health-promoting composition.

The Firmicutes to Bacteroidetes (F/B) ratio in the gut microbiome holds particular significance in the realm of obesity research, offering a valuable lens into the complex interplay between the microbiota and metabolic health. Studies consistently reveal an altered F/B ratio in individuals with obesity, characterized by an elevation in Firmicutes coupled with a reduction in Bacteroidetes. This imbalance is linked to pivotal shifts in energy metabolism, influencing the individual's propensity for weight gain and adiposity. Firmicutes, known for their proficiency in extracting energy from complex carbohydrates, contribute to increased caloric absorption and the storage of excess energy as fat. Conversely, the reduced abundance of Bacteroidetes may compromise the efficient utilization of dietary fiber, potentially impairing weight regulation. The perturbation of the F/B ratio becomes a distinctive marker of the metabolic landscape associated with obesity, serving as a crucial indicator of the microbiome's role in energy homeostasis. This intricate relationship between the F/B ratio and obesity not only provides insights into the underlying mechanisms of weight dysregulation but also opens avenues for targeted interventions. Harnessing a deeper understanding of the F/B ratio in the context of obesity holds promise for developing tailored strategies to modulate the gut microbiome, thereby offering innovative approaches to tackle the multifaceted challenges posed by obesity and its associated metabolic complications. In a personalized context, the predictive modeling of the present disclosure estimates the Firmicutes to Bacteroidetes (F/B) ratio for individual patients. For instance, an F/B ratio of 1.3 is predicted for the test patient. This personalized prediction can help to customize interventions based on an individual's predicted microbiome profile for obesity management.

Table 6 illustrates a list of representative microbes at genus level indicating beneficial and harmful bacteria for T2D (that is, for glucose control), and for Obesity (that is, for insulin sensitivity and weight management).

Table 6

| Genus | Role | Function | Impact |
|---|---|---|---|
| Agathobacter | Butyrate producer | Weight Management, Diabetes | |
| Akkermansia | glucose, insulin sensitivity and gut barrier integrity. | Weight Management | |
| Bacteroides | Metabolizing polysaccharides and oligosaccharides | Diabetes | |
| Bifidobacterium | Digest fibre | Weight Management | Beneficial |
| Gemmiger | Provide protection against IBD and liver diseases | | |

(continued)

| Genus | Role | Function | Impact |
|---|---|---|---|
| Oxalobacter | Maintain proper kidney health. | | |
| Prevotella | Breaks down polysaccharide | | |
| Slackia | It helps in lipid metabolism | | |
| Marvinbryantia | Butyrate producer | | |
| Monoglobus | It helps in controlling blood glucose level. | Diabetes | |
| Pseudoflavonifractor | obesity associated T2D development | Weight Management, Diabetes | |
| Rikenellaceae_RC9_gut_group | Involved in high fat diet induced obesity | Weight Management | Harmful |
| Turicibacter | Cause obesity and depression | | |

Similar lists for different disease conditions can be generated with the present disclosure to predict the disease status and intervention strategies for an individual leading to individualized or personalized probiotic formulation.

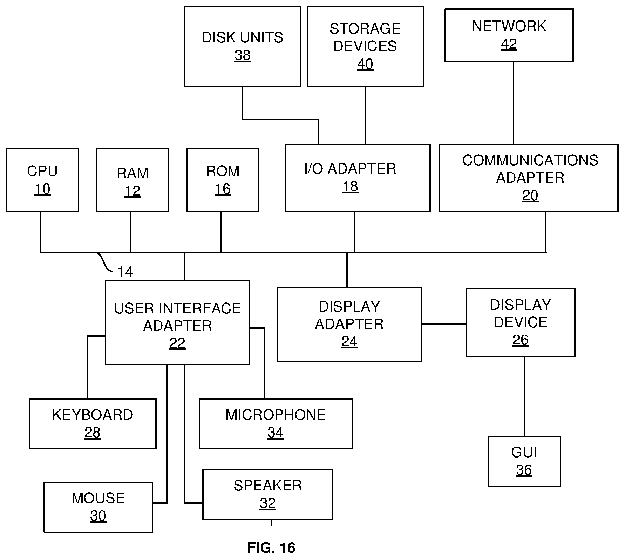

A representative hardware environment for practicing the embodiments herein is depicted in FIG. 16, with reference to FIGS. 1 through 15. This schematic drawing illustrates a hardware configuration of a microbiome determining server 108/computer system/ computing device in accordance with the embodiments herein. The system includes at least one processing device CPU 10 that may be interconnected via system bus 15 to various devices such as a random-access memory (RAM) 12, read-only memory (ROM) 16, and an input/output (I/O) adapter 18. The I/O adapter 18 can connect to peripheral devices, such as disk units 58 and program storage devices 50 that are readable by the system. The system can read the inventive instructions on the program storage devices 50 and follow these instructions to execute the methodology of the embodiments herein. The system further includes a user interface adapter 22 that connects a keyboard 28, mouse 50, speaker 52, microphone 55, and/or other user interface devices such as a touch screen device (not shown) to the bus 15 to gather user input. Additionally, a communication adapter 20 connects the bus 15 to a data processing network 52, and a display adapter 25 connects the bus 15 to a display device 26, which provides a graphical user interface (GUI) 56 of the output data in accordance with the embodiments herein, or which may be embodied as an output device such as a monitor, printer, or transmitter, for example.

The foregoing description of the specific embodiments will so fully reveal the general nature of the embodiments herein that others can, by applying current knowledge, readily modify and/or adapt for various applications without departing from the generic concept, and, therefore, such adaptations and modifications should be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the appended claims.

**Claims**

1. A system (100) for determining a microbiome profile from the metabolome of a host, comprising:

an analytical device (104) that is configured to analyze a sample and obtain spectral data of one or more metabolites present in the sample, wherein the sample is collected from the host on a sample collection device (102); and
a microbiome profile determination server (108) that is communicatively connected with the analytical device (104) and comprises

a memory; and
a processor in communication with the memory, wherein the processor is configured to:
receive the spectral data associated with the sample from the analytical device (104);

**characterized in that**,

the processor is further configured to generate metabolome data associated with the sample by detecting peaks associated with the one or more metabolites in the spectral data, and matching values of the peaks with theoretical peak values of known metabolites in a public database, wherein the peaks values comprise at least one of peak intensity, mass-to-charge (m/z), peak area, or full width at half maximum (FWHM) of peak, wherein the metabolome data comprises a plurality of metabolites in the sample with relative abundance; and predict, using a machine learning model (110), the microbiome profile of the host by extracting one or more features of the metabolome data and correlating the one or more features with learned association patterns to predict the microbiome profile, wherein the microbiome profile comprises at least one of phylum, genus or species level information on a microbial population associated with the host with relative abundance.

2. The system (100) as claimed in claim 1, wherein the host comprises at least one of human, animal, or microbes and the sample comprises at least one of whole blood, serum, plasma, faeces, saliva, skin tissues, microbial swabs from at least one of vaginal, oral or skin, or body fluids including tears, sweat and urine.

3. The system (100) as claimed in claim 1, wherein the analytical device (104) is a liquid chromatography tandem mass spectrometry (LC-MS/MS) analyzer that obtains the mass spectral data of the sample, wherein the mass spectral data comprises information about mass-to-charge (m/z) ratios of detected ions in the sample, relative abundance or intensity of the detected ions, and fragmentation pattern of ions, wherein the mass to charge (m/z) values of the peaks are matched with theoretical m/z values of known metabolites to generate the metabolome data.

4. The system (100) as claimed in claim 1, wherein the one or more features of the metabolome data are extracted based on the relative abundance of the plurality of metabolites in the sample, wherein the one or more features of the metabolome data comprise a presence of particular metabolite, a concentration of metabolites, ratios of certain metabolites, temporal dynamics of metabolite concentrations, and diversity indices of metabolites.

5. The system (100) as claimed in claim 1, wherein the machine learning model (110) is trained by

performing, using a regression model, a correlation analysis between metabolome data associated with historical blood samples and microbiome data in historical faecal samples to obtain association patterns relating to historical metabolites and the corresponding microbiome, wherein the association patterns are obtained based on high accuracy and high spearman correlation coefficient value; and
training the machine learning model (110) by mapping the historical metabolites to the corresponding microbiome based on the association patterns, thereby acquiring the learned association patterns that enable the machine learning model (110) to predict the microbiome profile.

6. The system (100) as claimed in claim 1, wherein the processor is configured to retrain the machine learning model (110) by mapping the metabolome data associated with the sample to the microbiome profile that is predicted.

7. The system (100) as claimed in claim 1, wherein the processor is configured to generate a microbiome report of the host based on the microbiome profile and send the microbiome report to a user device (112), wherein the microbiome report comprises at least one of phylum, genus or species level distribution of microbiome, information on harmful and helpful microbiomes with the abundance, and a firmicutes to bacteriodetes (F/B) ratio.

8. A method for determining a microbiome profile from the metabolome of a host, comprising:

collecting, using a sample collection device (102), a sample from the host;
obtaining, using an analytical device (104), spectral data of one or more metabolites present in the sample by analyzing the sample that is collected using the sample collection device (102);
receiving, by a processor of a microbiome profile determination server (108), the spectral data associated with the sample from the analytical device (104);

**characterized by**:

generating, by the processor, metabolome data associated with the sample by detecting peaks associated with the one or more metabolites in the spectral data, and matching values of the peaks with theoretical peak values of known metabolites in a public database, wherein the peaks values comprise at least one of peak intensity, mass-to-charge (m/z), peak area, or full width at half maximum (FWHM) of peak, wherein the metabolome data comprises a plurality of metabolites in the sample with relative abundance; and

predicting, by the processor, the microbiome profile of the host by extracting one or more features of the metabolome data and correlating the one or more features with learned association patterns to predict the microbiome profile using a machine learning model (110), wherein the microbiome profile comprises at least one of phylum, genus or species level information on a microbial population associated with the host with relative abundance.

9. The method as claimed in claim 8, wherein the one or more features of the metabolome data are extracted based on the relative abundance of the plurality of metabolites in the sample, wherein the one or more features of the metabolome data comprise a presence of particular metabolite, a concentration of metabolites, ratios of certain metabolites, temporal dynamics of metabolite concentrations, and diversity indices of metabolites.

10. The method as claimed in claim 8, comprising generating, by the processor, a microbiome report of the host based on the microbiome profile, wherein the microbiome report comprises phylum, genus or species level distribution of microbiome, information on harmful and helpful microbiomes with the abundance, and a firmicutes to bacteriodetes (F/B) ratio.


**Patentansprüche**

1. System (100) zur Bestimmung eines Mikrobiom-Profils aus dem Metabolom eines Wirts, umfassend:

eine Analysevorrichtung (104), die so konfiguriert ist, dass sie eine Probe analysiert und Spektraldaten von einem oder mehreren Metaboliten, die in der Probe vorhanden sind, erhält, wobei die Probe von dem Wirt mit einer Probensammelvorrichtung (102) gesammelt wird; und
einen Server (108) zur Bestimmung des Mikrobiom-Profils, der kommunikativ mit der Analysevorrichtung (104) verbunden ist und Folgendes umfasst
einen Speicher; und
einen Prozessor, der mit dem Speicher kommuniziert, wobei der Prozessor konfiguriert ist, um:

die mit der Probe verbundenen Spektraldaten von der Analysevorrichtung (104) zu empfangen;

**dadurch gekennzeichnet, dass**,

der Prozessor ferner so konfiguriert ist, dass er
mit der Probe assoziierte Metabolomdaten erzeugt, indem er mit dem einen oder den mehreren Metaboliten assoziierte Peaks in den Spektraldaten detektiert und Werte der Peaks mit theoretischen Peakwerten bekannter Metaboliten in einer öffentlichen Datenbank abgleicht, wobei die Peakwerte mindestens eines der folgenden Merkmale umfassen: Peakintensität, Masse-zu-Ladung (m/z), Peakfläche oder volle Breite bei halbem Maximum (FWHM) des Peaks, wobei die Metabolomdaten eine Mehrzahl von Metaboliten in der Probe mit relativer Abundanz umfassen; und
unter Verwendung eines Maschinenlernmodells (110), das Mikrobiom-Profil des Wirts vorhersagt durch Extrahieren eines oder mehrerer Merkmale der Metabolomdaten und Korrelieren des einen oder der mehreren Merkmale mit erlernten Assoziationsmustern, um das Mikrobiom-Profil vorherzusagen, wobei das Mikrobiom-Profil mindestens Informationen auf Phylum-, Gattungs- oder Artniveau über eine mit dem Wirt assoziierte mikrobielle Population mit relativer Abundanz umfasst.

2. System (100) nach Anspruch 1, wobei der Wirt mindestens eines von Mensch, Tier oder Mikroben umfasst und die Probe mindestens eines von Vollblut, Serum, Plasma, Fäkalien, Speichel, Hautgewebe, mikrobiellen Abstrichen aus mindestens einem der folgenden Bereiche: die Vagina, der Mund oder die Haut; oder Körperflüssigkeiten einschließlich Tränen, Schweiß und Urin umfasst.

3. System (100) nach Anspruch 1, wobei die Analysevorrichtung (104) ein Analysator von Flüssigkeitschromatographie mit Tandem-Massenspektrometrie (LC-MS/MS)-Kopplung ist, der die Massenspektraldaten der Probe erhält, wobei die Massenspektraldaten Informationen über die Masse-zu-Ladungs (m/z)-Verhältnisse der detektierten Ionen in der Probe, relative Abundanz oder Intensität der detektierten Ionen und Fragmentierungsmuster der Ionen umfassen, wobei die Masse-zu-Ladung (m/z)-Werte der Peaks mit theoretischen m/z-Werten bekannter Metaboliten abgeglichen werden, um die Metabolomdaten zu erzeugen.

4. System (100) nach Anspruch 1, wobei das eine oder die mehreren Merkmale der Metabolomdaten auf der Grundlage der relativen Abundanz der Vielzahl von Metaboliten in der Probe extrahiert werden, wobei das eine oder die mehreren Merkmale der Metabolomdaten ein Vorhandensein eines bestimmten Metaboliten, eine Konzentration von Metaboliten, Verhältnisse bestimmter Metaboliten, zeitliche Dynamik von Metabolitkonzentrationen und Diversitätsindizes von Metaboliten umfassen.

5. System (100) nach Anspruch 1, wobei das Maschinenlernmodell (110) trainiert wird durch

Durchführen einer Korrelationsanalyse unter Verwendung eines Regressionsmodells zwischen Metabolomdaten, die mit historischen Vollblutproben assoziiert sind, und Mikrobiomdaten in historischen Fäkalproben, um Assoziationsmuster zu erhalten, die sich auf historische Metaboliten und das entsprechende Mikrobiom beziehen, wobei die Assoziationsmuster auf der Grundlage einer hohen Genauigkeit und eines hohen Korrelationskoeffizientenwertes nach Spearman erhalten werden; und
Trainieren des Maschinenlernmodells (110) durch Zuordnung der historischen Metaboliten dem entsprechenden Mikrobiom auf der Grundlage der Assoziationsmuster, wodurch die gelernten Assoziationsmuster gewonnen werden, die es dem Maschinenlernmodell (110) ermöglichen, das Mikrobiom-Profil vorherzusagen.

6. System (100) nach Anspruch 1, wobei der Prozessor so konfiguriert ist, dass er das Maschinenlernmodell (110) neu trainiert, indem er ordnet die mit der Probe verbundenen Metabolomdaten dem Mikrobiom-Profil zu, das vorhergesagt wird.

7. System (100) nach Anspruch 1, wobei der Prozessor so konfiguriert ist, dass er einen Mikrobiom-Bericht des Wirts auf der Grundlage des Mikrobiom-Profils erzeugt und den Mikrobiom-Bericht an eine Benutzervorrichtung (112) sendet, wobei der Mikrobiom-Bericht mindestens eines der folgenden Elemente umfasst: Verteilung des Mikrobioms auf Phylum-, Gattungs- oder Artniveau, Informationen über schädliche und hilfreiche Mikrobiome mit der Abundanz und ein Firmicutes-Bacteroidetes (F/B)-Verhältnis.

8. Verfahren zur Bestimmung eines Mikrobiom-Profils aus dem Metabolom eines Wirts, umfassend:

Sammeln einer Probe des Wirts unter Verwendung einer Probensammelvorrichtung (102);
Erhalten von Spektraldaten eines oder mehrerer Metaboliten, die in der Probe vorhanden sind, unter Verwendung einer analytischen Vorrichtung (104) durch Analysieren der Probe, die unter Verwendung der Probensammelvorrichtung gesammelt wird (102);
Empfangen der der Probe zugeordneten Spektraldaten von der Analysevorrichtung (104) durch einen Prozessor eines Servers (108) zur Bestimmung des Mikrobiomprofils;

**gekennzeichnet durch**:

Erzeugen, durch den Prozessor, von Metabolomdaten, die mit der Probe verbunden sind, durch Erfassen von Peaks, die mit dem einen oder den mehreren Metaboliten in den Spektraldaten verbunden sind, und Abgleichen der Werte der Peaks mit theoretischen Peakwerten bekannter Metaboliten in einer öffentlichen Datenbank, wobei die Peakwerten mindestens eines von Peakintensität, Masse-zu-Ladung (m/z), Peakfläche oder volle Breite bei halbem Maximum (FWHM) des Peaks umfassen, wobei die Metabolomdaten eine Vielzahl von Metaboliten in der Probe mit relativer Abundanz umfassen; und
Vorhersagen das Mikrobiom-Profil des Wirts durch den Prozessor durch Extrahieren eines oder mehrerer Merkmale der Metabolomdaten und Korrelieren des einen oder der mehreren Merkmale mit gelernten Assoziationsmustern, um das Mikrobiom-Profil unter Verwendung eines Maschinenlernmodells (110) vorherzusagen, wobei das Mikrobiom-Profil mindestens Informationen auf Phylum-, Gattungs- oder Artniveau über eine mit dem Wirt assoziierte mikrobielle Population mit relativer Abundanz umfasst.

9. Verfahren nach Anspruch 8, wobei das eine oder die mehreren Merkmale der Metabolomdaten auf der Grundlage der relativen Abundanz der Vielzahl von Metaboliten in der Probe extrahiert werden, wobei das eine oder die mehreren Merkmale der Metabolomdaten ein Vorhandensein eines bestimmten Metaboliten, eine Konzentration von Metaboliten, Verhältnisse bestimmter Metaboliten, zeitliche Dynamik von Metabolitkonzentrationen und Diversitätsindizes von Metaboliten umfassen.

10. Verfahren nach Anspruch 8, umfassend das Erzeugen eines Mikrobiom-Berichts des Wirts durch den Prozessor auf der Grundlage des Mikrobiom-Profils, wobei der Mikrobiom-Bericht eines der folgenden Elemente umfasst: Verteilung des Mikrobioms auf Phylum-, Gattungs- oder Artniveau, Informationen über schädliche und hilfreiche Mikrobiome mit der Abundanz und ein Firmicutes-Bacteroidetes (F/B)-Verhältnis.

**Revendications**

1. Système (100) pour déterminer un profil de microbiome à partir du métabolome d'un hôte, comprenant :

   un dispositif d'analyse (104) configuré pour analyser un échantillon et obtenir des données spectrales d'un ou plusieurs métabolites présents dans l'échantillon, l'échantillon étant prélevé sur l'hôte à l'aide d'un dispositif de prélèvement d'échantillons (102) ; et
   un serveur de détermination du profil du microbiome (108) qui est connecté de manière communicative au dispositif d'analyse (104) et qui comprend
   une mémoire ; et
   un processeur en communication avec la mémoire, dans lequel le processeur est configuré pour :
   recevoir les données spectrales associées à l'échantillon du dispositif d'analyse (104) ;

   **caractérisé par le fait que** le processeur est en outre configuré pour

   générer des données de métabolome associées à l'échantillon en détectant des pics associés à l'un ou plusieurs métabolites dans les données spectrales, et en faisant correspondre les valeurs des pics avec les valeurs théoriques de pic de métabolites connus dans une base de données publique, les valeurs de pic comprenant au moins l'une des valeurs suivantes : l'intensité du pic, la masse sur charge (m/z), la surface du pic ou la pleine largeur à mi-hauteur (FWHM) du pic, les données de métabolome comprenant une pluralité de métabolites dans l'échantillon avec une abondance relative ; et
   prédire, à l'aide d'un modèle d'apprentissage par machine (110), le profil du microbiome de l'hôte en extrayant une ou plusieurs caractéristiques des données de métabolome et en corrélant la ou les caractéristiques avec des motifs d'association appris pour prédire le profil du microbiome, le profil du microbiome comprenant au moins des informations parmi celles au niveau du phylum, du genre ou de l'espèce sur une population microbienne associée à l'hôte avec une abondance relative.

2. Système (100) selon la revendication 1, dans lequel l'hôte comprend au moins un être humain, ou un animal ou des microbes et l'échantillon comprend au moins un des éléments suivants : sang total, sérum, plasma, fèces, salive, tissus cutanés, écouvillons microbiens provenant d'au moins un parmi le vagin, la bouche ou la peau, ou les fluides corporels, y compris les larmes, la sueur et l'urine.

3. Système (100) selon la revendication 1, dans lequel le dispositif d'analyse (104) est un analyseur de chromatographie en phase liquide par spectrométrie de masse (LC-MS/MS) en tandem qui obtient les données spectrales de masse de l'échantillon, dans lequel les données spectrales de masse comprennent des informations sur les rapports masse sur charge (m/z) des ions détectés dans l'échantillon, l'abondance ou l'intensité relative des ions détectés et le schéma de fragmentation des ions, les valeurs masse sur charge (m/z) des pics étant mises en correspondance avec les valeurs m/z théoriques de métabolites connus pour générer les données de métabolome.

4. Système (100) selon la revendication 1, dans lequel l'une ou plusieurs caractéristiques des données de métabolome sont extraites sur la base de l'abondance relative de la pluralité de métabolites dans l'échantillon, l'une ou plusieurs caractéristiques des données de métabolome comprenant la présence d'un métabolite particulier, une concentration de métabolites, des ratios de certains métabolites, la dynamique temporelle des concentrations de métabolites, et des indices de diversité des métabolites.

5. Système (100) selon la revendication 1, dans lequel le modèle d'apprentissage par machine (110) est formé en

   effectuant, à l'aide d'un modèle de régression, une analyse de corrélation entre les données de métabolome associées aux échantillons sanguins historiques et les données du microbiome dans des échantillons fécaux historiques afin d'obtenir des motifs d'association relatifs aux métabolites historiques et au microbiome correspondant, les motifs d'association étant obtenus sur la base d'une précision élevée et d'une valeur élevée du coefficient de corrélation de Spearman ; et
   formant le modèle d'apprentissage par machine (110) par le mappage des métabolites historiques vers le microbiome correspondant sur la base des motifs d'association, ce qui permet d'acquérir les motifs d'association appris qui permettent au modèle d'apprentissage par machine (110) de prédire le profil du microbiome.

6. Système (100) selon la revendication 1, dans lequel le processeur est configuré pour former à nouveau le modèle d'apprentissage par machine (110) par le mappage des données de métabolome associées à l'échantillon vers le profil du microbiome qui est prédit.

7. Système (100) selon la revendication 1, dans lequel le processeur est configuré pour générer un rapport sur le microbiome de l'hôte basé sur le profil du microbiome et envoyer le rapport sur le microbiome à un dispositif utilisateur (112), le rapport sur le microbiome comprenant au moins un des éléments suivants : la distribution du microbiome au niveau du phylum, du genre ou de l'espèce, des informations sur les microbiomes nuisibles et utiles avec l'abondance, et un rapport Firmicutes/Bactéroidetes (F/B).

8. Méthode de détermination d'un profil de microbiome à partir du métabolome d'un hôte, comprenant :

recueillir, à l'aide d'un dispositif de prélèvement d'échantillons (102), un échantillon provenant de l'hôte ;
obtenir, à l'aide d'un dispositif d'analyse (104), des données spectrales d'un ou plusieurs métabolites présents dans l'échantillon en analysant l'échantillon prélevé à l'aide du dispositif de prélèvement d'échantillons (102) ;
recevoir, par un processeur d'un serveur de détermination du profil du microbiome (108), les données spectrales associées à l'échantillon provenant du dispositif d'analyse (104) ;

**caractérisé par** :

générer, par le processeur, les données de métabolome associées à l'échantillon en détectant les pics associés à l'un ou plusieurs métabolites dans les données spectrales, et faire correspondre les valeurs des pics avec les valeurs théoriques des pics de métabolites connus dans une base de données publique, les valeurs des pics comprenant au moins une des valeurs suivantes : l'intensité du pic, le rapport masse sur charge (m/z), la surface du pic ou la pleine largeur à mi-hauteur (FWHM) du pic, les données de métabolome comprenant une pluralité de métabolites dans l'échantillon avec une abondance relative ; et
prédire, par le processeur, le profil du microbiome de l'hôte en extrayant une ou plusieurs caractéristiques des données de métabolome et en corrélant la ou les caractéristiques avec des motifs d'association appris pour prédire le profil du microbiome à l'aide d'un modèle d'apprentissage par machine (110), le profil du microbiome comprenant au moins des informations parmi celles au niveau du phylum, du genre ou de l'espèce sur une population microbienne associée à l'hôte avec une abondance relative.

9. Méthode selon la revendication 8, dans laquelle l'une ou plusieurs caractéristiques des données de métabolome sont extraites sur la base de l'abondance relative de la pluralité de métabolites dans l'échantillon, l'une ou plusieurs caractéristiques des données de métabolome comprenant la présence d'un métabolite particulier, une concentration de métabolites, des rapports de certains métabolites, une dynamique temporelle des concentrations de métabolites, et des indices de diversité des métabolites.

10. Méthode selon la revendication 8, comprenant la génération, par le processeur, d'un rapport sur le microbiome de l'hôte sur la base du profil du microbiome, le rapport sur le microbiome comprenant la distribution du microbiome au niveau du phylum, du genre ou de l'espèce, des informations sur les microbiomes nuisibles et utiles avec l'abondance, et un rapport Firmicutes/Bactéroidetes (F/B).

FIG. 1

FIG. 2

COLLECTING HISTORICAL BLOOD SAMPLES AND FAECAL SAMPLES FROM HISTORICAL HOSTS
302

ANALYSING HISTORICAL BLOOD SAMPLES USING AN ANALYTICAL DEVICE TO OBTAIN SPECTRAL DATA OF ONE OR MORE METABOLITES IN THE HISTORICAL BLOOD SAMPLES
304

ISOLATING DNA FROM THE FAECAL SAMPLES AND SUBJECTING THE DNA TO 16S RDNA METAGENOMIC SEQUENCING TO OBTAIN NEXT GENERATION DATA OF THE FAECAL SAMPLES
308

ANNOTATING THE SPECTRAL DATA OF THE HISTORICAL BLOOD SAMPLES AGAINST KNOWN METABOLITES IN THE PUBLIC DATABASE TO DETERMINE A METABOLOME DATA ASSOCIATED WITH THE HISTORICAL BLOOD SAMPLES
306

COMPARING AND ANNOTATING THE NEXT GENERATION DATA OF THE FAECAL SAMPLES AGAINST KNOWN METAGENOMIC DATABASES TO DETERMINE MICROBIOME DATA IN THE FAECAL SAMPLES
310

PERFORMING A CORRELATION ANALYSIS BETWEEN THE METABOLOME DATA ASSOCIATED WITH THE HISTORICAL BLOOD SAMPLES AND THE MICROBIOME DATA IN THE FAECAL SAMPLES TO IDENTIFY THE METABOLITES THAT ARE MOST STRONGLY ASSOCIATED WITH SPECIFIC MICROBIOME FEATURES, THEREBY OBTAINING ASSOCIATION PATTERNS
312

TRAINING THE MACHINE LEARNING MODEL WITH THE METABOLOME DATA OF THE HISTORICAL BLOOD SAMPLES AS INPUT, AND THE CORRESPONDING MICROBIOME DATA OF THE FAECAL SAMPLES AS OUTPUT, BASED ON THE ASSOCIATION PATTERNS TO OBTAIN A TRAINED MACHINE LEARNING MODEL
314

FIG. 3

COLLECTING A SAMPLE FROM THE HOST ON A SAMPLE COLLECTION
DEVICE
402

ANALYSING THE SAMPLE THAT IS COLLECTED ON THE SAMPLE
COLLECTION DEVICE USING AN ANALYTICAL DEVICE TO OBTAIN
SPECTRAL DATA OF ONE OR MORE METABOLITES PRESENT IN THE
SAMPLE
404

RECEIVING THE SPECTRAL DATA ASSOCIATED WITH THE SAMPLE BY A
PROCESSOR OF A MICROBIOME DETERMINING SERVER FROM THE
ANALYTICAL DEVICE THROUGH A NETWORK
406

GENERATING A METABOLOME DATA ASSOCIATED WITH THE SAMPLE, BY
THE PROCESSOR, BY DETECTING PEAKS ASSOCIATED WITH THE ONE OR
MORE METABOLITES IN THE SPECTRAL DATA, AND MATCHING VALUES
OF THE PEAKS WITH THEORETICAL PEAK VALUES OF KNOWN
METABOLITES IN A PUBLIC DATABASE
408

PREDICTING THE MICROBIOME PROFILE OF THE INDIVIDUAL, BY THE
PROCESSOR, BY EXTRACTING ONE OR MORE FEATURES OF THE
METABOLOME DATA AND CORRELATING THE ONE OR MORE FEATURES
WITH LEARNED ASSOCIATION PATTERNS TO PREDICT THE MICROBIOME
PROFILE USING A MACHINE LEARNING MODEL
410

GENERATING, BY THE PROCESSOR, A MICROBIOME REPORT OF THE
HOST BASED ON THE MICROBIOME PROFILE
412

**FIG. 4**

FIG. 5

NETWORK: CONTROL

FIG. 6A

NETWORK: OBESITY

FIG. 6B

FIG. 7

FIG. 8

FIG. 9

FIG. 10A

FIG. 10B

FIG. 10C

FIG. 11A

FIG. 11B

OBESITY - PHYLUM

FIG. 11C

FIG. 12A

FIG. 12B

FIG. 12C

FIG. 13A

FIG. 13B

FIG. 13C

PHYLUM-LEVEL DISTRUBUTION OF
MICROBIOME: TEST PATIENT

OTHERS

VERRUCOMICROBIA
7.0%

0.0%

24.6%

PROTEOBACTERIA
FIRMICUTES

18.2%

ACTINOBACTERIA
0.0%

50.3%

BACTEROIDETES

Firmicutes  Bacteriodetes  Actinobacteria  Proteobacteria  Verrumicrobia  Others

**FIG. 14**

1502

1504

33  31

Harmful  Helpful

**FIG. 15**

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAN SHUO et al.** A metabolomics pipeline for the mechanistic interrogation of the gut microbiome. *NATURE*, 14 July 2021, vol. 595, 415-420